# EUROPEAN PATENT APPLICATION

(11) **EP 4 821 917 A1**
(43) Date of publication of application: **14.10.2026**
(21) Application number: 24899886.6
(22) Date of filing: 04.12.2024
(51) Int. Cl.: C07C 237/22, C07C 233/76, C07D 209/02, A61K 31/167, A61P 35/00

(54) **PROLIFERATING CELL NUCLEAR ANTIGEN INHIBITOR, AND PREPARATION METHOD THEREFOR, INTERMEDIATE THEREOF, PHARMACEUTICAL COMPOSITION THEREOF AND USE THEREOF**

(30) Priority: 05.12.2023 CN 202311667122; 03.01.2024 CN 202410008207; 28.11.2024 CN 202411726617
(71) Applicant: Du Xinyun, Redwood City, CA 94065 (US); Huang, Qiang, Suzhou, Jiangsu 215627 (CN)
(72) Inventor: DU, Jessica Xinyun, Redwood City California 94065 (US)
(74) Representative: Bandpay & Greuter
(86) International application number: PCT/CN2024/136935
(87) International publication number: WO 2025/119248

(57) **Abstract**

A compound represented by formula (I) as a PCNA inhibitor, or a pharmaceutically acceptable salt thereof, or a deuterated compound thereof, a preparation method therefor, an intermediate compound thereof, a pharmaceutical composition thereof and the use thereof. The PCNA inhibitor and the pharmaceutical composition comprising same can be used for treating cancers, comprising lung cancer, melanoma, colon cancer, rectal cancer, prostate cancer, ovarian cancer, leukemia, etc with PCNA overexpression.

## Description

### TECHNICAL FIELD

The present disclosure relates to a proliferating cell nuclear antigen inhibitor, a preparation method therefor, a pharmaceutical composition thereof, and use thereof for treatment of cancer such as lung cancer, melanoma, colon cancer, rectal cancer, prostate cancer, ovarian cancer, and leukemia.

### BACKGROUND

Proliferating Cell Nuclear Antigen (abbreviated as PCNA) is a highly conserved protein that plays a crucial role in both gene replication and gene repair. In cancer cells, the expression level of PCNA is typically significantly increased because these cells undergo more frequent DNA replication to maintain their rapid proliferation. Furthermore, PCNA is also involved in DNA repair and damage responses of tumor cells. PCNA can serve as not only a cancer biomarker but also an important target for cancer therapy (Oncogene 2023, 42, 613).

Chinese patent (CN108348512) discloses that AOH-1996 and its analogs can target and bind to PCNA, showing a significant anti-cancer activity in various cancer cell lines and cancer models, while having fewer side effects on normal cells.

Among PCNA-targeting compounds, ATX-101 exhibits an antitumor activity in a variety of tumor cells with minimal impact on healthy cells, making it the first compound to enter clinical trials and show antitumor efficacy (Oncogene 2023, 42, 541).

Given the limited studies on PCNA inhibitors, there is a significant clinical need. Therefore, it is essential to discover and develop safer and more effective PCNA inhibitors to improve the therapeutic efficacy against cancer.

### SUMMARY

The present inventors have found through studies that modifying the methoxybenzene ring of AOH-1996 with a bicyclic ring or further replacing the oxygen (O) between the two benzene rings with sulfur (S) has a significant effect on its antitumor activity. For example, as shown in the structural formulae below, MTB-1956 obtained by introducing cyclopentyl ether at the 3,4-position of the phenyl group in AOH-1996 shows three folds higher inhibitory activity against human acute promyelocytic leukemia cells (HL-60) than AOH-1996, whereas MTB-1962 obtained by introducing an N-S five-membered heterocycle ring at the 3,4-position shows a 32-fold decrease in activity. Through extensive screening, the present inventors have obtained a more potent PCNA inhibitor that improves the therapeutic effect against cancer. Furthermore, deuteration of the hydrogen atoms in AOH-1996 could improve its pharmacokinetic properties.

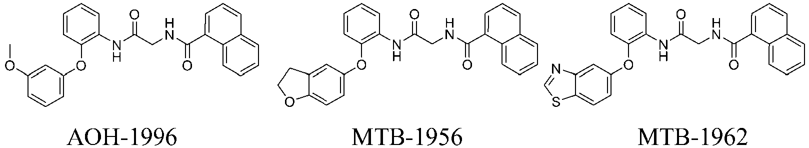

In view of this, the present disclosure provides a new PCNA inhibitor that is useful for treatment of cancer, such as lung cancer, melanoma, colon cancer, rectal cancer, prostate cancer, ovarian cancer, and leukemia, particular those with PCNA overexpression.

The present disclosure provides a compound represented by formula (I), or a pharmaceutically acceptable salt thereof, or a deuterated compound thereof, 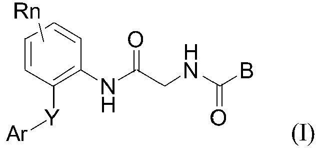 wherein,
B is substituted or unsubstituted naphthyl, substituted or unsubstituted quinolinyl, or substituted or unsubstituted isoquinolinyl; optionally, B is substituted or unsubstituted naphthyl; optionally, B is naphthyl; optionally, substituents in the substituted naphthyl, the substituted quinolinyl, and the substituted isoquinolinyl are each independently C₁₋₆ alkyl, C₃₋₇ cycloalkyl, C₆₋₁₀ aryl, halogen, or halogenated C₁₋₆ alkyl; optionally, the substituents are each independently methyl, ethyl, n-propyl, isopropyl, cyclopropyl, phenyl, F, CI, Br, I, or -CF₃;
each R is independently C₁₋₆ alkyl, C₃₋₇ cycloalkyl, substituted or unsubstituted C₆₋₁₀ aryl, substituted or unsubstituted 5- to 10-membered heteroaryl containing 1 to 3 heteroatoms selected from N, O, and S, -NH₂, -CN, halogen (such as F, Cl, Br, I), -OH, or -OR', wherein R' is C₁₋₆ alkyl, C₃₋₇ cycloalkyl, or substituted or unsubstituted C₆₋₁₀ aryl; optionally, the alkyl is methyl, ethyl, n-propyl, or isopropyl; the cycloalkyl is cyclopropyl; the substituents in the substituted aryl and the substituted heteroaryl are each independently C₁₋₆ alkyl, C₃₋₇ cycloalkyl, C₆₋₁₀ aryl, halogen, or halogenated C₁₋₆ alkyl; optionally, the substituents are each independently methyl, ethyl, n-propyl, isopropyl, cyclopropyl, phenyl, F, CI, Br, I, or -CF₃;
n is an integer selected from 0 to 3; optionally, n is 0;
Y is O or S; preferably, Y is O; preferably, Y is S;
Ar is carbocyclic ring-fused phenyl, heterocyclic ring-fused phenyl, carbocyclic ring-fused heteroaryl, or heterocyclic ring-fused heteroaryl; optionally, Ar is carbocyclic ring-fused phenyl or heterocyclic ring-fused phenyl; the carbocyclic ring is a saturated or non-aromatic unsaturated 5-membered or 6-membered carbocyclic ring, the heterocyclic ring is a 5-membered or 6-membered heterocyclic ring containing 1 or 2 N atoms, 1 or 2 O atoms, or 1 or 2 S atoms on a saturated or non-aromatic unsaturated ring, and the heteroaryl is a 6-membered heteroaryl containing 1 or 2 N atoms on the ring; optionally, Ar is heterocyclic ring-fused phenyl, and the heterocyclic ring is a 5- or 6-membered heterocyclic ring containing 1 or 2 O atoms on a saturated or non-aromatic unsaturated ring;
the carbocyclic ring-fused phenyl, the heterocyclic ring-fused phenyl, the carbocyclic ring-fused heteroaryl, and the heterocyclic ring-fused heteroaryl are optionally each independently substituted by one or more substituents, and the substituents are each independently C₁₋₆ alkyl, C₃₋₇ cycloalkyl, OH, CN, NH₂, mono- or di-C₁₋₆ alkylamino, halogen, or halogenated C₁₋₆ alkyl; optionally, the carbocyclic ring-fused phenyl, the heterocyclic ring-fused phenyl, the carbocyclic ring-fused heteroaryl, and the heterocyclic ring-fused heteroaryl are optionally each independently substituted by one or more substituents, and the substituents are each independently methyl, ethyl, n-propyl, isopropyl, cyclopropyl, -F, -Cl, -Br, -I, -CH₂CF₃, or -CF₃; optionally, the substituents are each independently -CH₃, -F, -Cl, or -CF₃.

Optionally, in the compound represented by formula (I), or the pharmaceutically acceptable salt thereof, or the deuterated compound thereof as described above,
Ar is selected from the following groups: 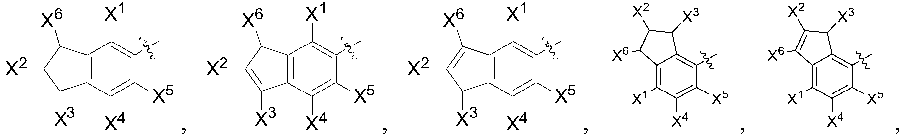 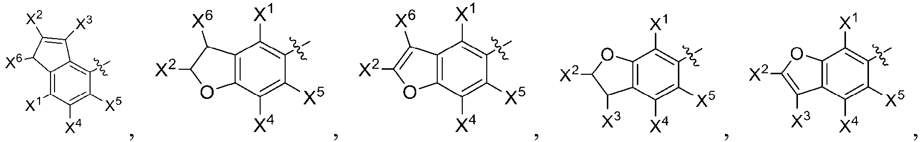 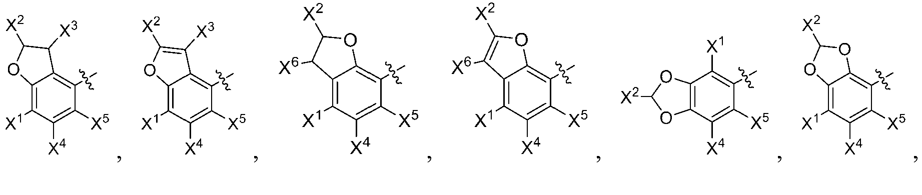 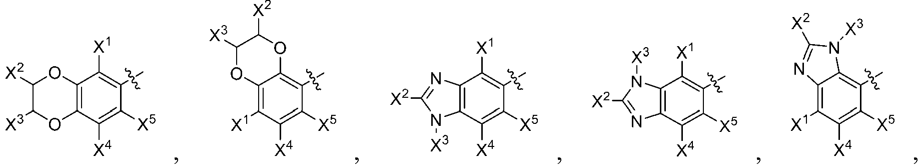 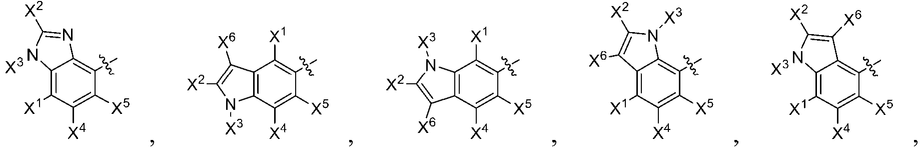 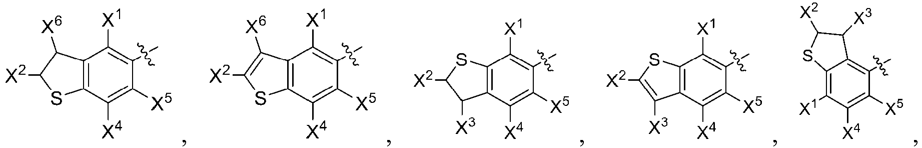 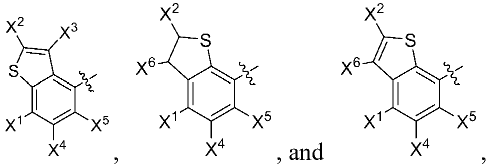
wherein X¹, X², X³, X⁴, X⁵, and X⁶ are each independently H, C₁₋₆ alkyl (such as methyl, ethyl, n-propyl, or isopropyl), C₃₋₇ cycloalkyl (such as cyclopropyl), OH, CN, NH₂, halogen (such as Cl, Br, I, or F), or halogenated C₁₋₆ alkyl; optionally, X¹, X⁴, and X⁵ are each independently H, methyl, ethyl, n-propyl, isopropyl, cyclopropyl, -F, -Cl, -Br, -I, -CH₂CF₃, or -CF₃, preferably X¹ and/or X⁴ and/or X⁵ is hydrogen; optionally, X¹, X⁴, and X⁵ are each independently H, -CH₃, -F, -Cl, or -CF₃, preferably X¹ and/or X⁴ and/or X⁵ is hydrogen; optionally, X², X³, and X⁶ are each independently H, methyl, ethyl, n-propyl, isopropyl, cyclopropyl, -F, -Cl, -Br, -I, -CH₂CF₃, or -CF₃, preferably X² and/or X³ and/or X⁶ is hydrogen; optionally, X², X³, and X⁶ are each independently H or -CH₃, preferably two or all of X², X³, and X⁶ are hydrogen.

Optionally, in the compound represented by formula (I), or the pharmaceutically acceptable salt thereof, or the deuterated compound thereof as described above,
Ar is selected from the following groups: 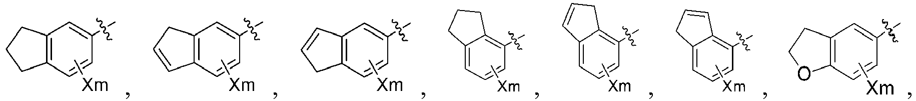 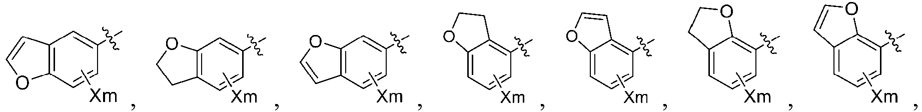 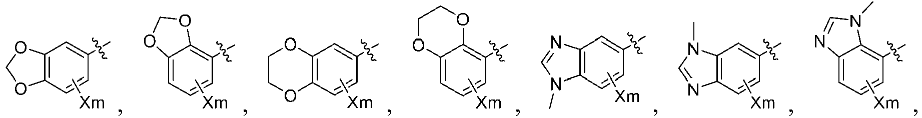 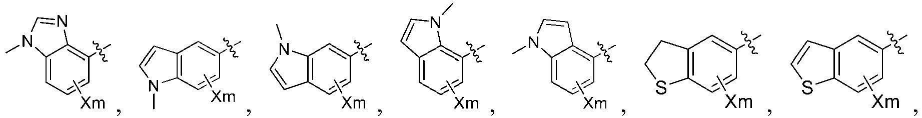 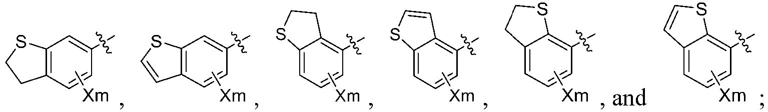
wherein each X is independently C₁₋₆ alkyl (such as methyl, ethyl, n-propyl, or isopropyl), C₃₋₇ cycloalkyl (such as cyclopropyl), OH, CN, NH₂, halogen (such as Cl, Br, I, or F) or halogenated C₁₋₆ alkyl; optionally, each X is independently methyl, ethyl, n-propyl, isopropyl, cyclopropyl, F, Cl, Br, I, -CH₂CH₃, or -CF₃; optionally, each X is independently -CH₃, -F, -Cl, or -CF₃;
m is an integer of from 0 to 3; optionally, m is 0, 1 or 2.

Optionally, in the compound represented by formula (I), or the pharmaceutically acceptable salt thereof, or the deuterated compound thereof as described above,
the compound represented by formula (I) is selected from the following compounds: 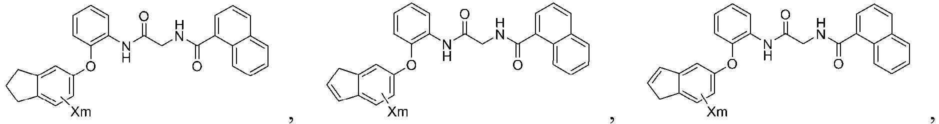 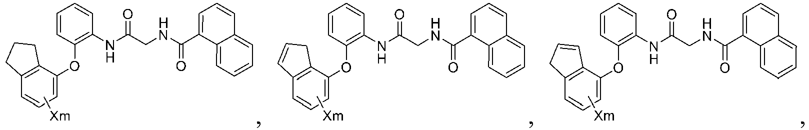 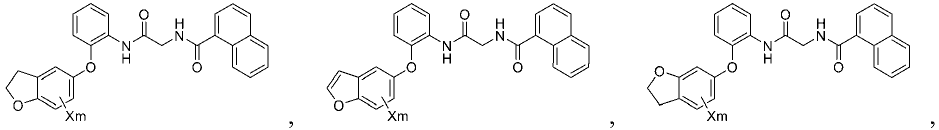 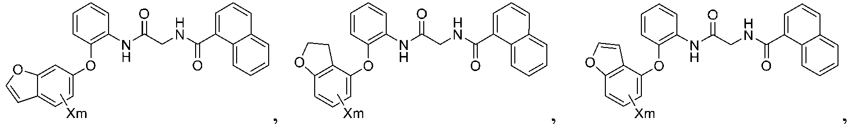 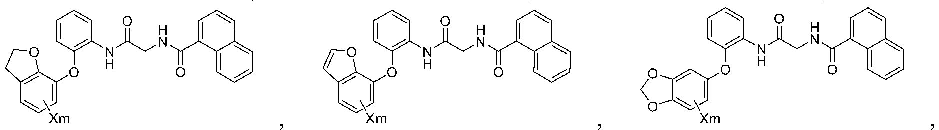 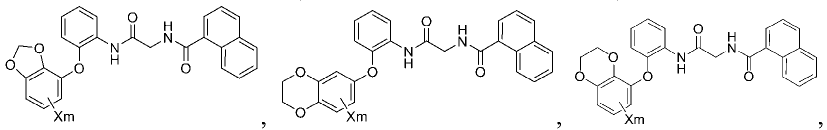 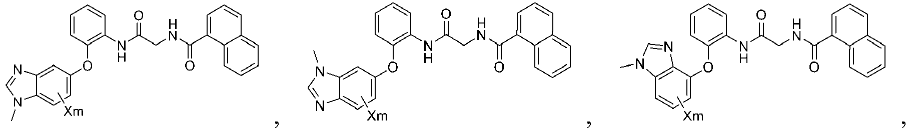 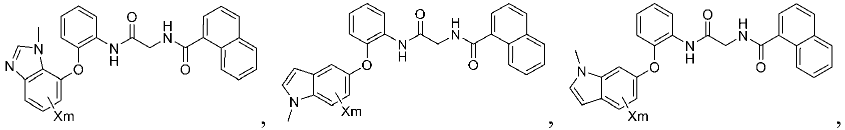 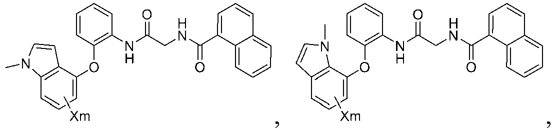 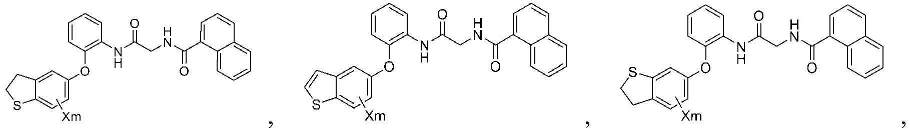 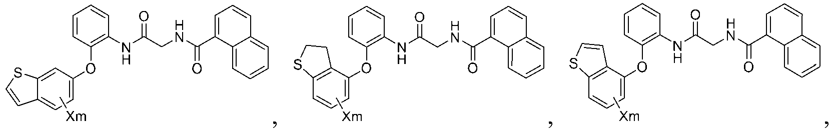 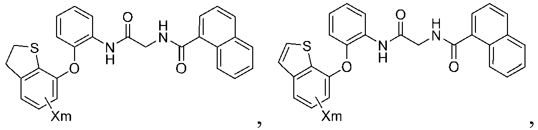 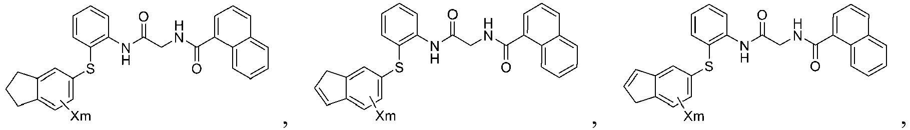 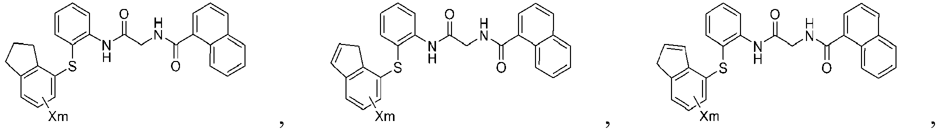 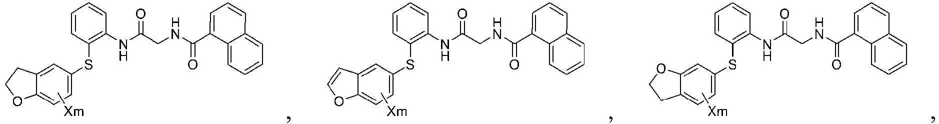 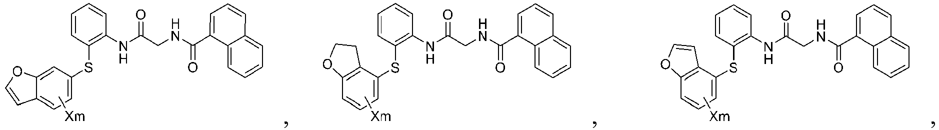 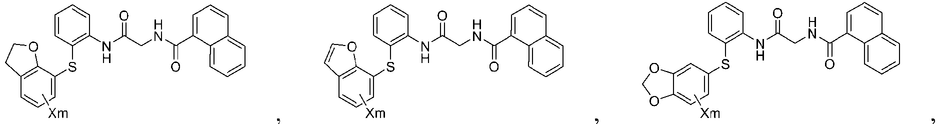 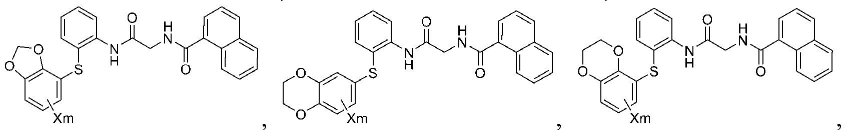 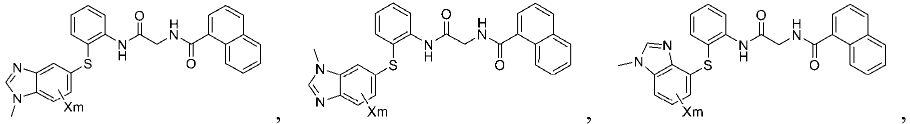 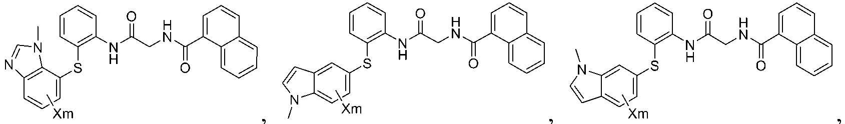 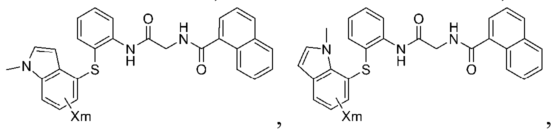 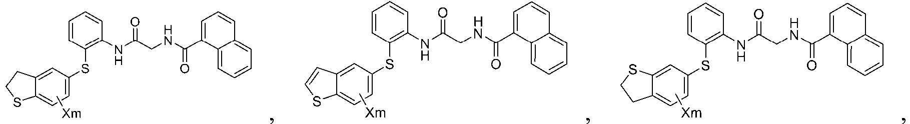 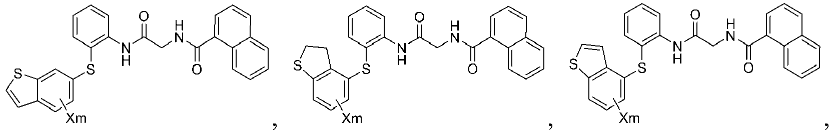 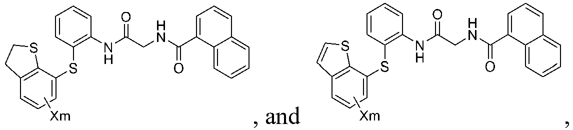
wherein each X is independently C₁₋₆ alkyl (such as methyl, ethyl, n-propyl, or isopropyl), C₃₋₇ cycloalkyl (such as cyclopropyl), -OH, -CN, -NH₂, halogen (such as Cl, Br, I, or F), or halogenated C₁₋₆ alkyl; optionally, each X is independently methyl, ethyl, propyl, n-propyl, isopropyl, cyclopropyl, -F, -Cl, -Br, -I, -CH₂CH₃, or -CF₃; optionally, each X is independently -CH₃, -F, -Cl, or -CF₃;
m is an integer of from 0 to 3; optionally, m is 0, 1 or 2.

Optionally, in the compound represented by formula (I), or the pharmaceutically acceptable salt thereof, or the deuterated compound thereof as described above,
the compound represented by formula (I) or the deuterated compound thereof is selected from the following compounds: 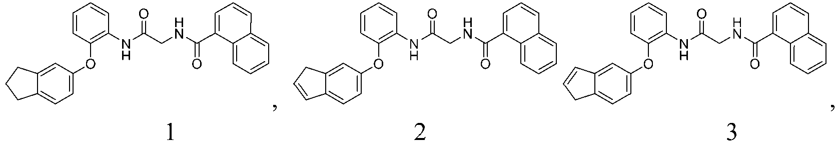 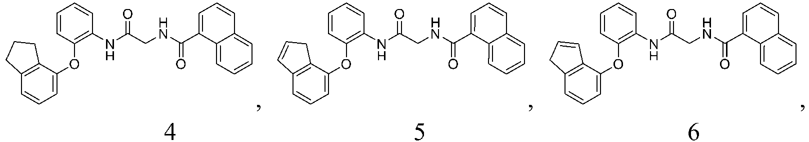 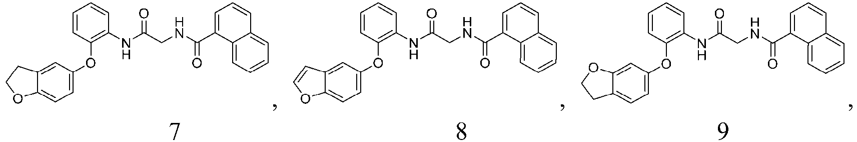 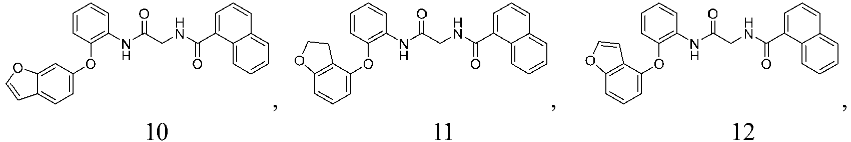 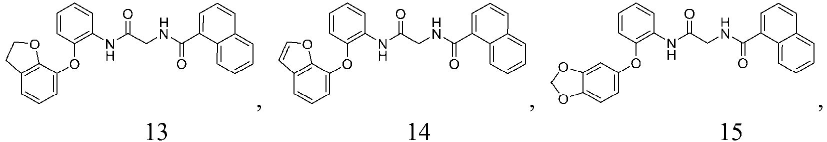 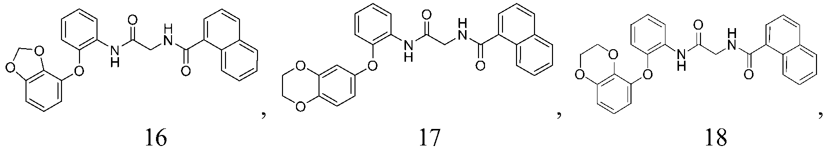 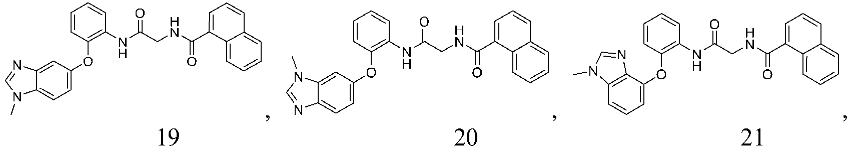 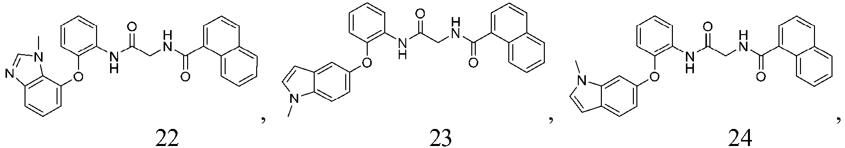 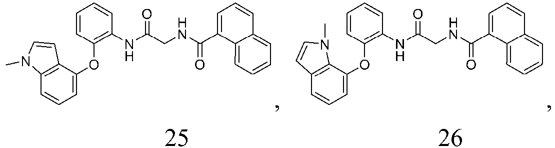 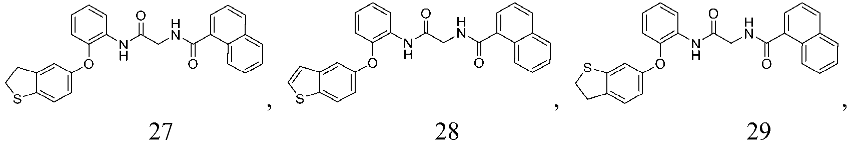 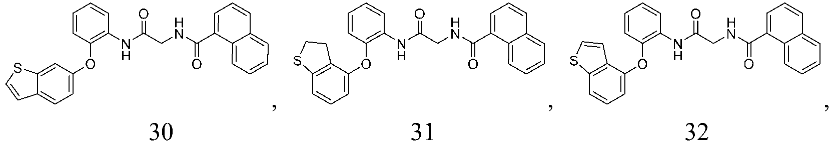 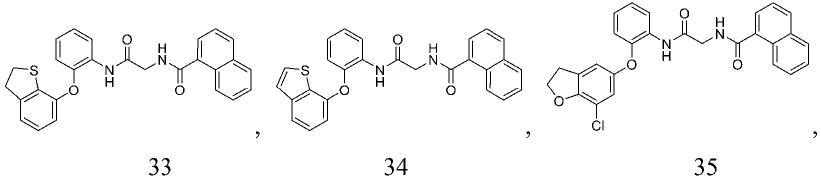 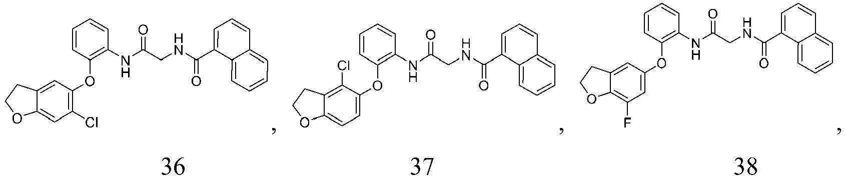 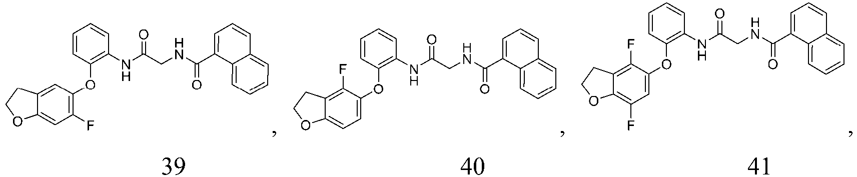 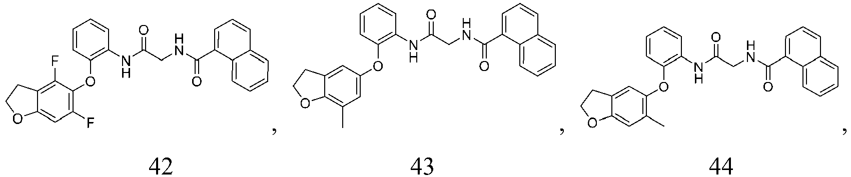 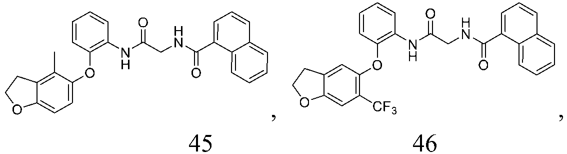 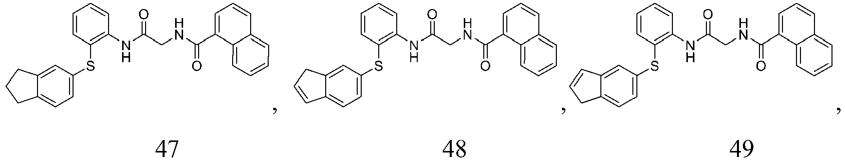 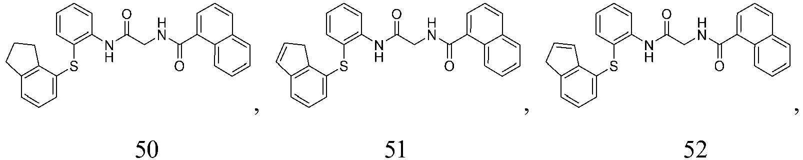 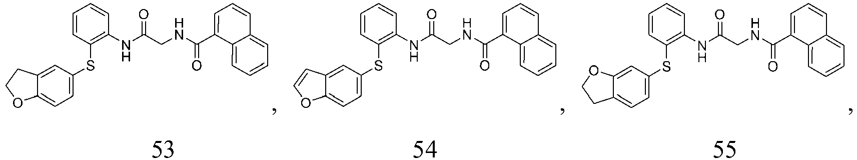 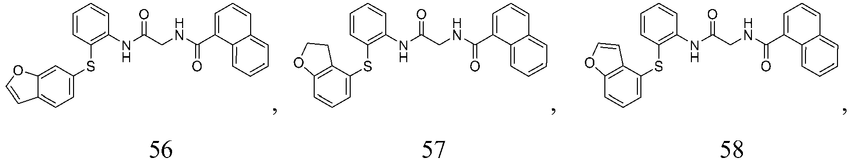 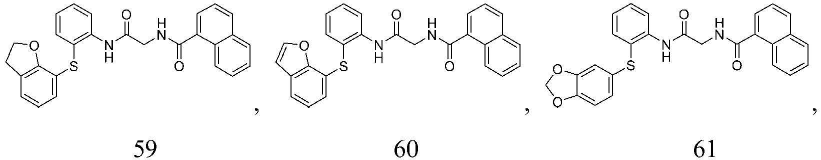 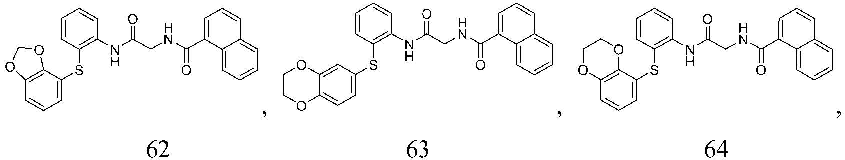 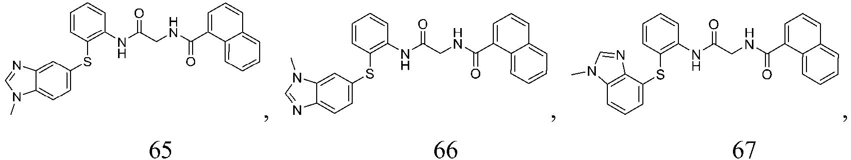 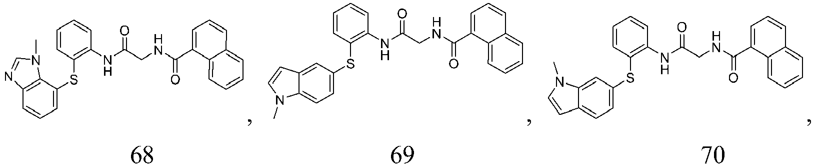 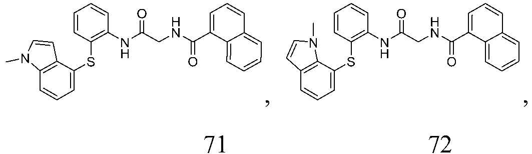 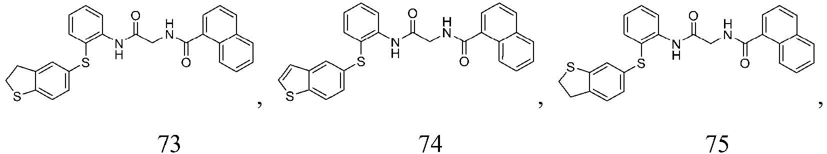 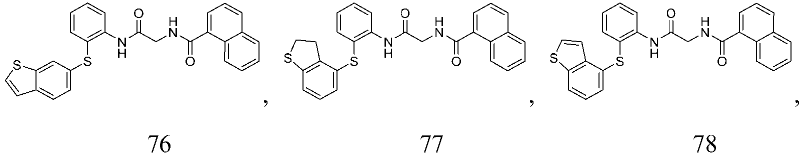 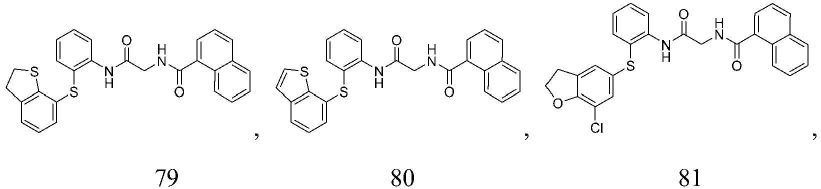 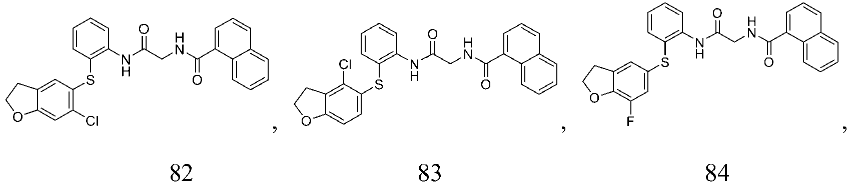 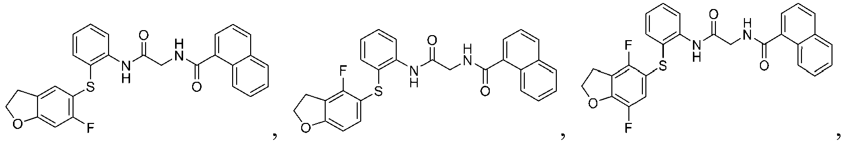 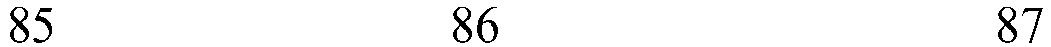 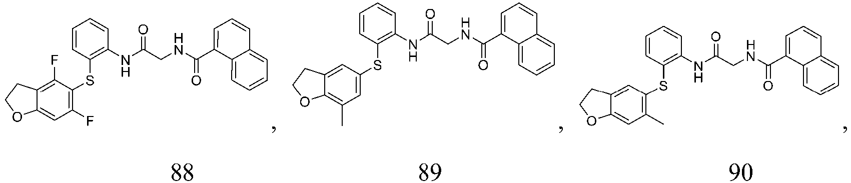 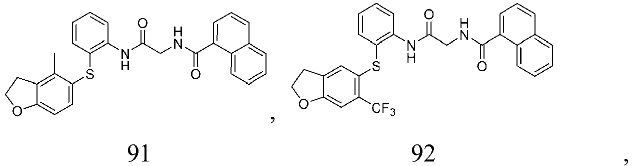 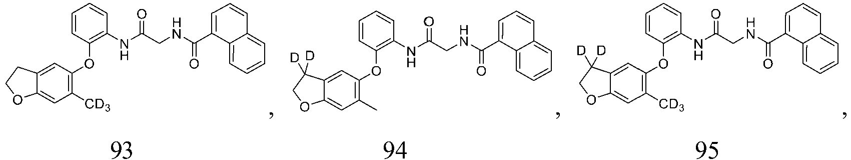 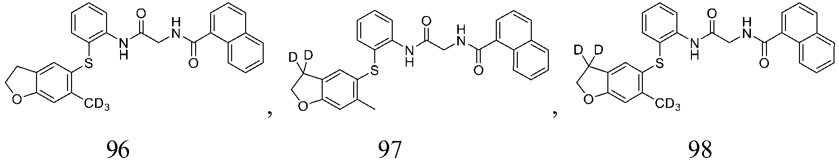 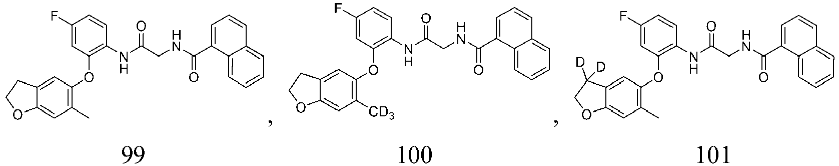 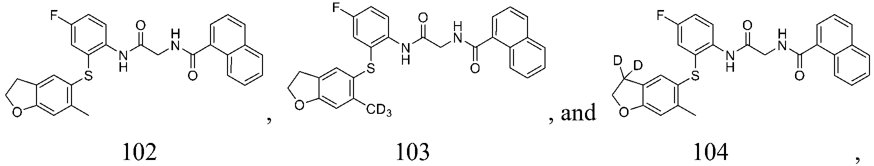 particularly preferably compound 44, compound 93, compound 99 or compound 100.

Optionally, in the compound represented by formula (I), or the pharmaceutically acceptable salt thereof, or the deuterated compound thereof as described above, the pharmaceutically acceptable salt is a salt formed by the compound represented by formula (I) or the deuterated compound thereof with an acid, the acid includes an inorganic acid or an organic acid; optionally, the inorganic acid includes hydrochloric acid, hydrobromic acid, sulfuric acid, nitric acid, phosphoric acid, or carbonic acid; optionally, the organic acid includes formic acid, acetic acid, propionic acid, oxalic acid, malonic acid, succinic acid, phthalic acid, fumaric acid, maleic acid, lactic acid, malic acid, citric acid, tartaric acid, carbonic acid, picric acid, methanesulfonic acid, ethanesulfonic acid, benzenesulfonic acid, or p-toluenesulfonic acid.

The pharmaceutically acceptable salt may be prepared by a conventional method in the art.

The compound represented by formula (I), the pharmaceutically acceptable salt thereof, or the deuterated compound thereof according to the present disclosure includes various optical isomers thereof, such as resonance tautomers, racemates, enantiomers, and diastereomers.

The compound represented by formula (I), the pharmaceutically acceptable salt thereof, or the deuterated compound thereof according to the present disclosure may be present in such forms as amorphous, crystalline, and solvate compounds (e.g., hydrates).

The present disclosure further provides a method for preparing a compound represented by formula (I), or a pharmaceutically acceptable salt thereof, or a deuterated compound thereof, wherein the method comprises the following reaction route:

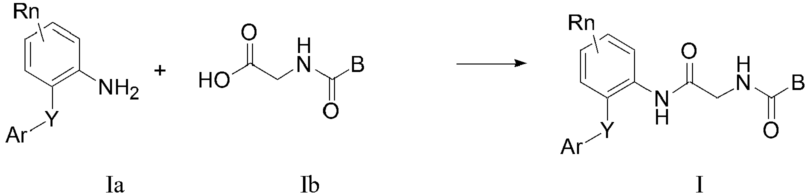

In the above structural formulas, Ar, Y, R, n, and B are as defined above.

The reaction process of this reaction route is as follows: Compound Ia as a precursor of aniline and compound Ib as a glycine derivative undergo a condensation reaction in an organic solvent (e.g., DMF) under the action of a condensing agent (polypeptide condensing agent (HATU)) and a catalyst (e.g., N,N-diisopropylethylamine (DIPEA)) to obtain the target product compound I or a deuterated compound thereof.

The present disclosure further involves the intermediate compounds below. Among them, compound 44-37 may be used to synthesize compound 44; compound 93-38 may be used to synthesize compound 93; compound 99-39 may be used to synthesize compound 99; compound 100-40 may be used to synthesize compound 100; compound 44-37-6 may be used to synthesize compound 44-37, compound 44, compound 99-39, and compound 99; and compound 93-98-5 may be used to synthesize compound 93-38, compound 93, compound 100-40, and compound 100.

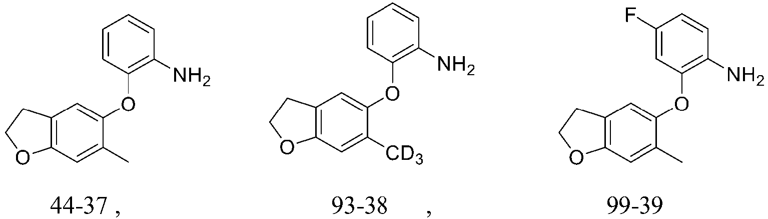

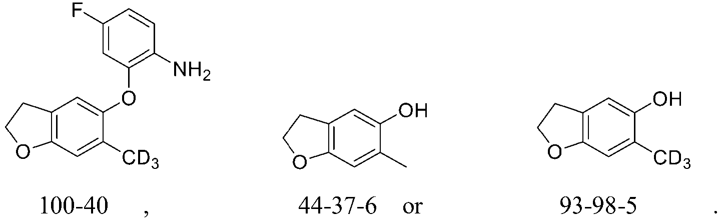

The present disclosure further provides a pharmaceutical composition, comprising one or more of the above-mentioned compound represented by formula (I), or the pharmaceutically acceptable salt thereof, or the deuterated compound thereof according to the present disclosure, and optionally a pharmaceutically acceptable excipient. Optionally, the pharmaceutical composition further comprises an anticancer drug in addition to the compound represented by formula (I), or the pharmaceutically acceptable salt thereof, or the deuterated compound thereof; preferably, the anticancer drug comprises a chemotherapeutic drug, or/and a targeted drug, or/and a radiopharmaceutical. Preferably, the anticancer drug includes, but is not limited to, chemotherapeutic drugs such as cyclophosphamide, antimetabolite drugs such as fluorouracil, gemcitabine, and capecitabine, antibiotics such as doxorubicin, platinum-based anticancer drugs such as carboplatin, cisplatin, and oxaliplatin, phytopharmaceutical drugs such as irinotecan, paclitaxel, and taxotere, targeted drugs such as afatinib, trastuzumab, imatinib, panitumumab, ramucirumab, and osimertinib, immune preparations such as pembrolizumab, nivolumab, and Keytruda, ADC-based drugs such as gemtruzumab, trastuzumab emtansine, sacituzumab tirumotecan, trastuzumab deruxtecan, and Enhertu, bispecific antibody drugs such as PD-1/CTLA-4 bispecific antibody cadonilimab and PD-(L)1/VEGF bispecific antibody ivonescimab, and radiopharmaceuticals such as lutetium[177Lu] vipivotide tetraxetan injection (Pluvicto) and Lutathera.

The present disclosure further provides use of the above-mentioned compound of formula (I), or pharmaceutically acceptable salt thereof, or deuterated compound thereof according to the present disclosure, or the above-mentioned pharmaceutical composition in the preparation of a PCNA inhibitor.

The present disclosure further provides use of the above-mentioned compound of formula (I), or pharmaceutically acceptable salt thereof, or deuterated compound thereof according to the present disclosure, or the above-mentioned pharmaceutical composition in the preparation of a medicament for preventing or treating cancer; wherein optionally, the cancer includes lung cancer, melanoma, colon cancer, rectal cancer, prostate cancer, ovarian cancer, and leukemia; particularly cancer with PCNA overexpression.

The present disclosure further provides a method for preventing or treating cancer, comprising administering, to a subject in need thereof, a prophylactically or therapeutically effective amount of the above-mentioned compound represented by formula (I), or pharmaceutically acceptable salt thereof, or deuterated compound thereof, or the above-mentioned pharmaceutical composition.

The compound represented by formula (I), or pharmaceutically acceptable salt thereof, or deuterated compound thereof described herein, or a pharmaceutical composition containing the compound represented by formula (I), or pharmaceutically acceptable salt thereof, or deuterated compound thereof described herein may be used alone or in combination with additional drugs. Appropriate combinations may produce a synergistic effect, improve the therapeutic effect on the cancer described above, or reduce the toxic and side effects of drugs, or may reduce the toxic and side effects while producing a synergistic effect. The additional drugs include, but are not limited to, a chemotherapeutic drug, a targeted drug, a radiopharmaceutical, and the like. Preferably, the anticancer drug includes, but is not limited to, chemotherapeutic drugs such as cyclophosphamide, antimetabolite drugs such as fluorouracil, gemcitabine, and capecitabine, antibiotics such as doxorubicin, platinum-based anticancer drugs such as carboplatin, cisplatin, and oxaliplatin, phytopharmaceutical drugs such as irinotecan, paclitaxel, and taxotere, targeted drugs such as afatinib, trastuzumab, imatinib, panitumumab, ramucirumab, and osimertinib, immune preparations such as pembrolizumab, nivolumab, and Keytruda, ADC-based drugs such as gemtruzumab, trastuzumab emtansine, sacituzumab tirumotecan, trastuzumab deruxtecan, and Enhertu, bispecific antibody drugs such as PD-1/CTLA-4 bispecific antibody cadonilimab and PD-(L)1/VEGF bispecific antibody ivonescimab, radiopharmaceuticals such as lutetium[177Lu] vipivotide tetraxetan injection (Pluvicto) and Lutathera.

### DETAILED DESCRIPTION

Specific embodiments of the present disclosure will be described in detail below. It should be appreciated that the specific embodiments described herein are only intended to illustrate the present disclosure by way of example, rather than to limit the present disclosure.

### Preparation of Compounds

### Preparation Example 1. Preparation of Compound 8 (MTB-1956)

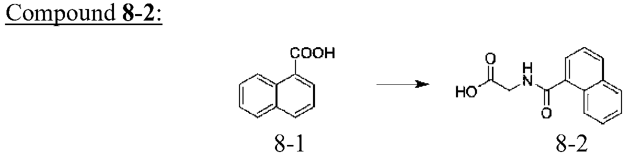

Compound **8-1** (40 g, 232.32 mmol) was refluxed in SOCl₂ (150 mL) for 2 h, distilled to remove SOCl₂, and co-evaporated with 1,4-dioxane (20 mL) two times. The residue was dissolved in 1,4-dioxane (100 mL). NaOH (18.6 g, 464.64 mmol) was dissolved in water (232 mL). Thereafter, glycine (17.4 g, 232.32 mmol) was added to an aqueous sodium hydroxide solution, dissolved, and then added dropwise to the above reaction solution at 0°C. The resulting reaction solution was warmed to the room temperature and reacted for 16 h, and extracted with EA (200 mL). The aqueous phase was adjusted with 5N HCl to pH 2. A solid precipitated, filtered, washed with water, and dried to yield compound **8-2** (41.8 g, 79%) as a light yellow solid.

¹H NMR (400 MHz, DMSO) δ 12.71 (s, 1H), 8.88 (t, *J =* 6.0 Hz, 1H), 8.36 - 8.28 (m, 1H), 8.04 (d, *J =* 8.2 Hz, 1H), 8.02 - 7.95 (m, 1H), 7.64 (dd, *J =* 7.0, 1.3 Hz, 1H), 7.61- 7.51 (m, 3H), 4.00 (d, *J =* 6.0 Hz, 2H).

m/z(ESI) [M+H]⁺=230.1.

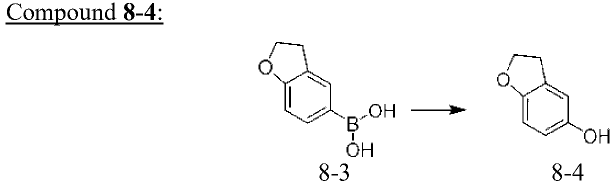

Compound **8-3** (5 g, 30.49 mmol) was dissolved in THF (60 mL), NaHCO₃ (5.1 g, 60.98 mmol) was added, and H₂O₂ (30%, 6.2 mL, 60.98 mmol) was added dropwise at 0°C. The mixture was reacted at room temperature for 16 h. A saturated sodium bisulfite solution was added to quench the reaction. The reaction solution was extracted with EA (200 mL). The organic phase was dried over anhydrous sodium sulfate, filtered, concentrated, and subjected to silica gel column chromatography (methanol:CH₂Cl₂=1:30-1:20) to yield compound **8-4** (4.1 g, 99%) as a white solid.

¹H NMR (400 MHz, CDCl₃) δ 6.76 - 6.69 (m, 1H), 6.64 (d, *J =* 8.5 Hz, 1H), 6.57 (dd, *J* = 8.5, 2.6 Hz, 1H), 4.73 (s, 1H), 4.54 (t, *J =* 8.6 Hz, 2H), 3.16 (t, *J* = 8.6 Hz, 2H).

*m*/*z*(ESI)[M-H]⁻=135.1.

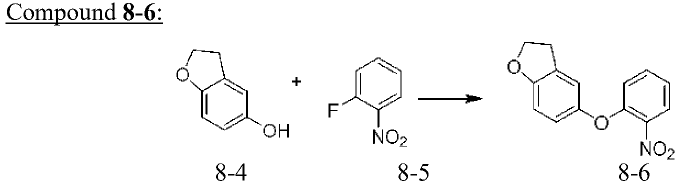

Compound **8-5** (3.5 g, 25.09 mmol), compound **8-4** (4.1 g, 30.11 mmol), and K₂CO₃ (3.8 g, 27.60 mmol) were added to DMF (50 mL), reacted at 100°C for 16 h under nitrogen protection, and cooled. The reaction solution was extracted with EA (100 mL). The organic phase was dried over anhydrous sodium sulfate, filtered, concentrated, and purified by silica gel column chromatography (EA:petroleum ether=1:5-1:3) to yield compound **8-6** (6.4 g, 83%) as a white solid.

m/z(ESI) [M+Na]⁺=279.9.

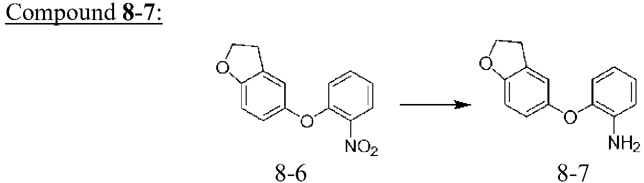

Compound **8-6** (6.4 g, 24.9 mmol) was dissolved in a mixed solvent of EA (50 mL) and MeOH (50 mL), 0.6 g of 10% Pd/C was added, and hydrogen was charged. The mixture was reacted at room temperature for 16 h. The reaction solution was filtered through diatomite, and washed with EA. The filtrate was concentrated to yield compound **8-7** (5.6 g, 99%) as a grey oily product.

¹H NMR (400 MHz, CDCl₃) δ 6.98 - 6.89 (m, 1H), 6.88 - 6.83 (m, 1H), 6.81 (dd, *J= 7.8,* 1.6 Hz, 1H), 6.79 - 6.74 (m, 2H), 6.74 - 6.68 (m, 2H), 4.57 (t, *J =* 8.6Hz, 2H), 3.86 (s, 2H), 3.18 (t, *J =* 8.6 Hz, 2H).

m/z(ESI) [M+H]⁺=228.1.

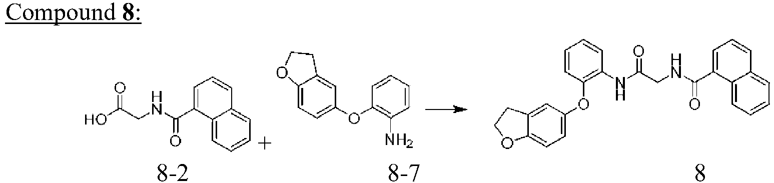

Compound **8-7** (2 g, 8.8 mmol), compound **8-2** (2.4 g, 10.56 mmol), and HATU (5.0g, 13.2 mmol) were added to DMF (25 mL), and then DIPEA (4.6 mL, 26.4 mmol) was added dropwise. The mixture was reacted at room temperature for 16 h under nitrogen protection. The reaction solution was extracted with EA (100 mL), dried over anhydrous sodium sulfate, filtered, concentrated, purified by silica gel column chromatography (EA:petroleum ether=2:10-1:2) and then recrystallized (EA/petroleum ether) to yield compound **8** (2.2 g, 57%) as an off-white solid.

¹H NMR (400 MHz, CDCl₃) δ 8.53 (s, 1H), 8.39 (dd, *J* = 7.9, 1.1 Hz, 1H), 8.34 (d, *J* = 8.4 Hz, 1H), 7.92 (d, *J* = 8.3 Hz, 1H), 7.89 - 7.82 (m, 1H), 7.64 (dd, *J =* 7.0, 1.0 Hz, 1H), 7.48 (dtd, *J =* 16.6, 6.9, 1.3 Hz, 2H), 7.39 (dd, *J* = 8.1, 7.2 Hz, 1H), 7.02 (dtd, *J =* 24.5, 7.6, 1.4 Hz, 2H), 6.90-6.80 (m,2H), 6.80-670 (m, 3H), 4.58 (t, *J* = 8.7 Hz, 2H), 4.39 (d, *J* = 5.5 Hz, 2H), 3.15 (t, *J= 8.7* Hz, 2H).

*m*/*z*(ESI)[M+H]⁺=439.1.

### Preparation Example 2. Preparation of Compound 9 (MTB-1959)

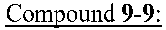

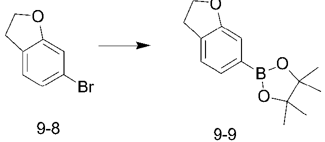

Compound **9-8** (5 g, 25.12 mmol), bis(pinacolato)diboron (7.7 g, 30.14 mmol), potassium acetate (7.4 g, 75.36 mmol), and Pd(dppf)Cl₂ (919 mg, 1.26 mmol) were added to dioxane (50 mL), reacted at 90°C for 6 h under nitrogen protection, and cooled to room temperature. EA (100 mL) and water (20 mL) were added and mixed. The reaction solution was filtered through diatomite. The filtrate was extracted. The organic phase was dried over anhydrous sodium sulfate, filtered, concentrated, and purified by silica gel column chromatography (EA:petroleum ether=1:5-1:3) to yield compound **9-9** (6 g, 97%) as a light yellow solid.

¹H NMR (400 MHz, CDCl₃) δ 7.32 (d, *J =* 7.4 Hz, 1H), 7.22-7.19 (m, 2H), 4.54 (t, *J =* 8.7 Hz, 2H), 3.21 (t, *J=* 8.7 Hz, 2H), 1.33 (s, 12H).

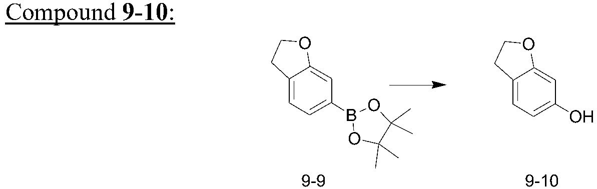

Compound **9-9** (6 g, 24.38 mmol) was dissolved in THF (50 mL), NaHCO₃ (4.1 g, 48.76 mmol) was added, and then H₂O₂ (30%, 5 mL, 48.76 mmol) was added dropwise at 0°C. The mixture was reacted at room temperature for 3 h. A saturated sodium bisulfite solution was added to quench the reaction. The reaction solution was extracted with EA (100 mL), dried over anhydrous sodium sulfate, filtered, concentrated, and purified by silica gel column chromatography (methanol:CH₂Cl₂=1:30-1:20) to yield compound **9-10** (3.23 g, 97%) as a colorless oily product.

¹H NMR (400 MHz, CDCl₃) δ 7.08 - 6.88 (m, 1H), 6.39 - 6.19 (m, 2H), 4.87 (s, 1H), 4.57 (t, *J =* 8.6 Hz, 2H), 3.12 (t, *J =* 8.6 Hz, 2H).

*m*/*z*(ESI)[M+H]⁺=137.1.

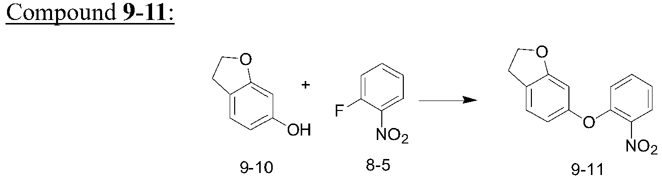

Compound **8-5** (3.0 g, 21.57 mmol), compound **9-10** (3.23 g, 23.72 mmol), and K₂CO₃ (3.23 g, 23.72 mmol) were added to DMF (40 mL), reacted at 110°C for 16 h under nitrogen protection, and cooled. The reaction solution was extracted with EA (100 mL), dried over anhydrous sodium sulfate, filtered, concentrated, and purified by silica gel column chromatography (EA:petroleum ether=1:10-1:3) to yield compound **9-11** (5.08 g, 92%) as a yellow solid.

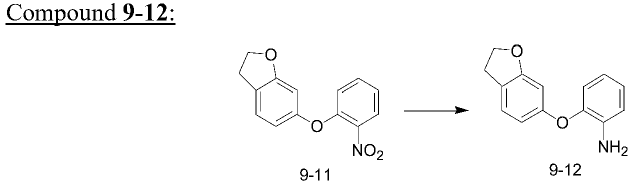

Compound **9-11** (5.08 g, 19.7 mmol) was dissolved in a mixed solvent of EA (40 mL) and MeOH (40 mL), 10% Pd/C (0.5 g) was added, and hydrogen was charged. The mixture was reacted at room temperature for 16 h. The reaction solution was filtered through diatomite, and washed with EA. The filtrate was concentrated to yield compound **9-12** (4.4 g, 98%) as a yellow oily product.

¹H NMR (400 MHz, CDCl₃) δ 7.07 (dd, *J =* 7.2, 1.1 Hz, 1H), 7.02 - 6.93 (m, 1H), 6.88 (dd, *J =* 8.0, 1.4 Hz, 1H), 6.81 (dd, *J =* 7.9, 1.5 Hz, 1H), 6.71 (ddd, *J* = 8.0, 7.5, 1.6 Hz, 1H), 6.51 - 6.42 (m, 2H), 4.59 (t, *J =* 8.7 Hz, 2H), 3.78 (s, 2H), 3.16 (t, *J =* 8.4 Hz, 2H).

*m*/*z*(ESI)[M+H]⁺=228.1.

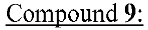

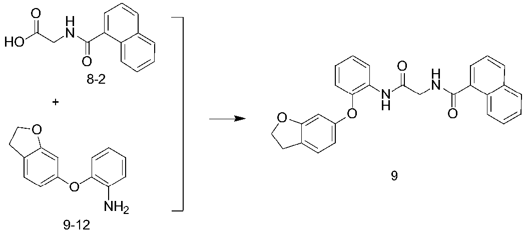

Compound **9-12** (2 g, 8.8 mmol), compound **8-2** (2.4 g, 10.56 mmol), and HATU (5.0g, 13.2 mmol) were added to DMF (25 mL). Thereafter, DIPEA (4.6 mL, 26.4 mmol) was added dropwise to the reaction solution, and reacted at room temperature for 16 h under nitrogen protection. The reaction solution was extracted with EA (100 mL), dried over anhydrous sodium sulfate, filtered, concentrated, purified by silica gel column chromatography (EA:petroleum ether=1:10-1:2), and recrystallized (EA/petroleum ether) to yield compound **9** (1.8 g, 47%) as a yellow solid.

¹H NMR (400 MHz, CDCl₃) δ 8.49 - 8.36 (m, 2H), 8.36 - 8.27 (m, 1H), 7.91 (d, *J =* 8.2 Hz, 1H), 7.89 - 7.80 (m, 1H), 7.63 (d, *J =* 6.4 Hz, 1H), 7.57 - 7.44 (m, 2H), 7.44 - 7.34 (m, 1H), 7.15-7.0 (m, 3H), 6.89 (d, *J* = 8.1 Hz, 2H), 6.55 - 6.40 (m, 2H), 4.59 (t, *J* = 8.7 Hz, 2H), 4.35 (d, *J* = 5.4 Hz, 2H), 3.15 (t, *J* = 8.7 Hz, 2H).

m/z(ESI) [M+H]⁺=439.1.

### Preparation Example 3. Preparation of Compound 15 (MTB-1958)

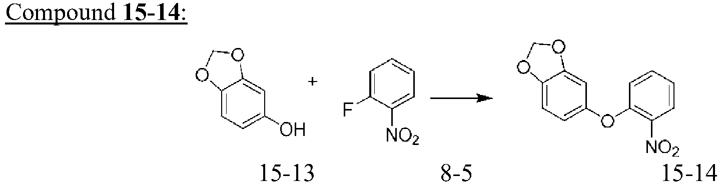

Compound **8-5** (5.0 g, 35.44 mmol), compound **15-13** (5.4 g, 38.98 mmol), and K₂CO₃ (5.4 g, 38.98 mmol) were added to DMF (50 mL), reacted at 100°C for 16 h under nitrogen protection, and cooled to room temperature. The reaction solution was extracted with EA (200 mL), dried over anhydrous sodium sulfate, filtered, concentrated, and purified by silica gel column chromatography (EA:petroleum ether=1:10-1:3) to yield compound **15-14** (9.0 g, 98%) as a yellow solid.

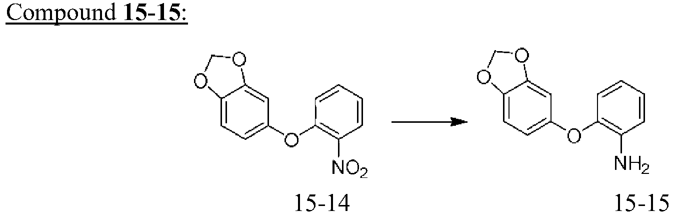

Compound **15-14** (9 g) as obtained above was dissolved in a mixed solvent of EA (80 mL) and MeOH (40 mL), 10% Pd/ (0.9 g) was added, and hydrogen was charged, and reacted at room temperature for 16 h. The reaction solution was filtered through diatomite, and washed with EA. The filtrate was concentrated to yield compound **15-15** (8 g, 98%) as a brown oily product.

¹H NMR (400 MHz, CDCl₃) δ 6.98 - 6.90 (m, 1H), 6.81 (dt, *J= 7.8,* 1.5 Hz, 2H), 6.76 - 6.66 (m, 2H), 6.56 (d, *J =* 2.4 Hz, 1H), 6.44 (dd, *J* = 8.4, 2.5 Hz, 1H), 5.95 (s, 2H), 3.82 (s, 2H).

m/z(ESI) [M+H]⁺=230.1.

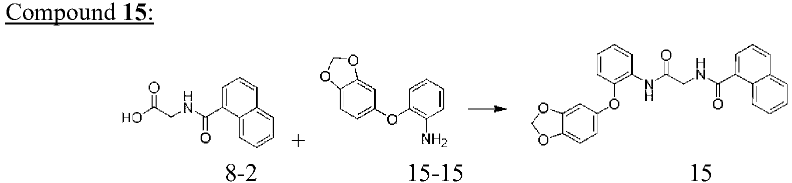

Compound **15-15** (2 g, 8.72 mmol), compound **8-2** (2.4 g, 10.46 mmol), and HATU (5.0 g, 13.08 mmol) were added to DMF (25 mL), and then DIPEA (4.6 mL, 26.16 mmol) was added dropwise. The mixture was reacted at room temperature for 16 h under nitrogen protection. The reaction solution was extracted with EA (100 mL), dried over anhydrous sodium sulfate, filtered, concentrated, purified by silica gel column chromatography (EA:petroleum ether=1:10-1:2), and recrystallized (EA/petroleum ether) to yield compound 15 (0.7 g, 18%) as an off-white solid.

¹H NMR (400 MHz, CDCl₃) δ 8.50 (s, 1H), 8.39 (d, *J =* 6.9 Hz, 1H), 8.36 - 8.29 (m, 1H), 7.92 (d, *J =* 8.2 Hz, 1H), 7.88 - 7.81 (m, 1H), 7.64 (dd, *J* = 7.0, 1.0 Hz, 1H), 7.58 - 7.45 (m, 2H), 7.40 (dd, *J =* 8.1, 7.2 Hz, 1H), 7.08 (td, *J* = 7.8*,* 1.3 Hz, 1H), 7.01 (td, *J* = 7.8*,* 1.4 Hz, 1H), 6.87 (t, *J =* 5.0 Hz, 1H), 6.81 (dd, *J =* 8.1, 1.2 Hz, 1H), 6.73 (d, *J =* 8.4 Hz, 1H), 6.56 (d, *J =* 2.4 Hz, 1H), 6.47 (dd, *J* = 8.4, 2.4 Hz, 1H), 5.96 (s, 2H), 4.38 (d, *J =* 5.5 Hz, 2H).

*m*/*z*(ESI)[M+H] ⁺=441.2.

### Preparation Example 4. Preparation of Compound 17 (MTB-1961):

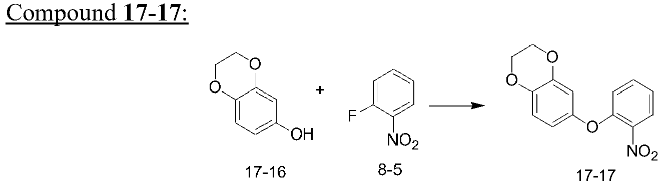

Compound **8-5** (3.0 g, 21.26 mmol), compound **17-16** (3.6 g, 23.39 mmol), and K₂CO₃ (3.23 g, 23.39 mmol) were added to DMF (50 mL), reacted at 100°C for 16 h under nitrogen protection, and cooled to room temperature. The reaction solution was extracted with EA (100 mL), dried over anhydrous sodium sulfate, filtered, concentrated, and subjected to silica gel column chromatography (EA:petroleum ether=1:10-1:3) to yield compound **17-17** (5.8 g, 99%) as a yellow solid.

*m*/*z*(ESI)[M+H]⁺=274.0.

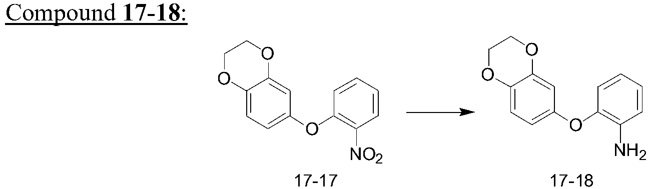

Compound **17-17** (5.8 g, 21.21 mmol) was dissolved in a mixed solvent of EA (50 mL) and MeOH (40 mL), 10% Pd/C (0.6 g) was added, and hydrogen was charged. The mixture was reacted at room temperature for 16 h. The reaction solution was filtered through diatomite, and washed with EA. The filtrate was concentrated to yield compound **17-18** (5.0 g, 97%) as a red oily product.

*m*/*z*(ESI)[M+H]⁺=244. 1.

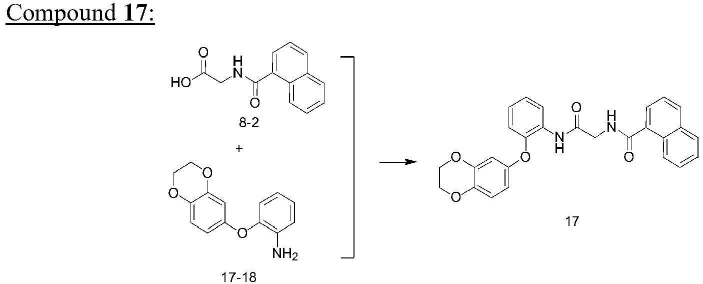

Compound **17-18** (2 g, 8.22 mmol), compound **8-2** (2.26 g, 9.86 mmol), and HATU (4.7 g, 12.33 mmol) were added to DMF (25 mL), and then DIPEA (4.3 mL, 24.66 mmol) was added dropwise. The mixture was reacted at room temperature for 16 h under nitrogen protection. The reaction solution was extracted with EA (100 mL), dried over anhydrous sodium sulfate, filtered, concentrated, purified by silica gel column chromatography (EA:petroleum ether=1:10-1:2), and recrystallized (EA/petroleum ether) to yield compound 17 (0.5 g, 13%) as a yellow solid.

¹H NMR (400 MHz, CDCl₃) δ 8.47 (s, 1H), 8.38 (d, *J* = 7.5 Hz, 1H), 8.36 - 8.29 (m, 1H), 7.91 (d, *J* = 8.2 Hz, 1H), 7.87 - 7.81 (m, 1H), 7.64 (d, *J* = 7.0 Hz, 1H), 7.57 - 7.44 (m, 2H), 7.40 (t, *J* = 7.7 Hz, 1H), 7.07 (td, *J* = 7.8, 1.2 Hz, 1H), 7.04 - 6.98 (m, 1H), 6.90 (s, 1H), 6.83 (dd, *J* = 11.1, 4.9 Hz, 2H), 6.58 (d, *J* = 2.8 Hz, 1H), 6.52 (dd, *J =* 8.8, 2.8 Hz, 1H), 4.36 (d, *J=* 5.4 Hz, 2H), 4.23 (s, 4H).

*m*/*z*(ESI)[M+H]⁺=455.1.

### Preparation Example 5. Preparation of Compound 20 (MTB-1965):

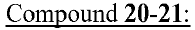

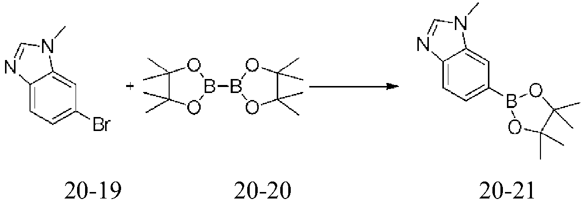

Compound **20-19** (5.0 g, 23.7 mmol) and compound 20-20 (7.2 g, 28.4 mmol) were placed in a round-bottom flask, and potassium acetate (6.98 g, 71.1 mmol) and PdCl₂(dppf) (0.87 g, 1.19 mmol) were added, and dissolved in 1,4-dioxane (60 mL). The mixture was reacted with stirring at 90°C for 3 h. Water was added at 0°C to quench the reaction. The reaction solution was extracted with EA (100 mL), filtered through diatomite, and washed two times with saturated saline, dried over anhydrous sodium sulfate, filtered, concentrated, and purified by silica gel column chromatography (EA:petroleum ether=1:10-1:3) to yield compound **20-21** (2.36 g, 38%) as a yellow solid.

¹H NMR (400 MHz, CDCl3) δ 7.91 (s, 2H), 7.82 (d, *J =* 8.2 Hz, 1H), 7.75 (d, *J =* 8.2 Hz, 1H), 3.87 (s, 3H), 1.39 (s, 12H).

*m*/*z*(ESI)[M+H]⁺=259.

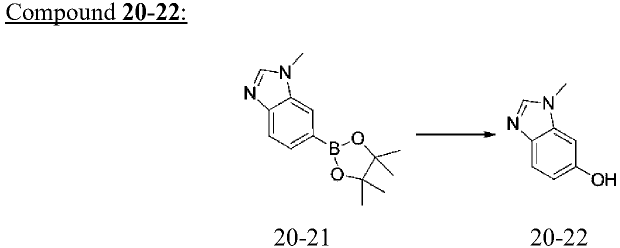

Compound **20-21** (2.36 g, 9.14 mmol) was placed in a round-bottom flask, and dissolved in THF (25 mL). Sodium bicarbonate (1.54 g, 18.29 mmol) was added. Hydrogen peroxide (30%, 1.87 mL, 18.3 mmol) was added dropwise at 0°C. The mixture was reacted with stirring at room temperature for 3 h. Saturated sodium bisulfite was added to quench the reaction. The reaction solution was concentrated, co-evaporated with methanol two times, and purified by silica gel column chromatography (methanol:CH₂Cb=1:30-1:20) to yield compound **20-22** (1.0 g) as a white solid, which was directly used for the next step.

¹H NMR (400 MHz, DMSO) δ 9.27 (s, 1H), 7.94 (s, 1H), 7.40 (d, *J =* 8.6 Hz, 1H), 6.82 (d, *J =* 2.2 Hz, 1H), 6.69 (dd, *J =* 2.2, 8.6 Hz, 1H), 3.72 (s, 3H).

*m*/*z*(ESI)[M+H] ⁺=149.

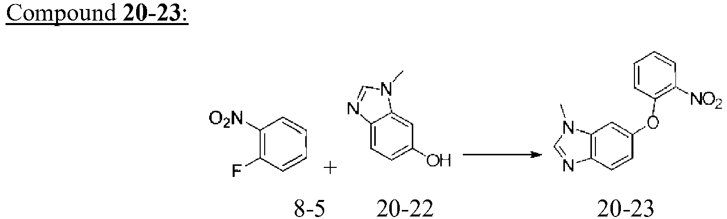

Compound **20-22** (1.0 g, 6.82 mmol) and compound **8-5** (875 mg, 6.2 mmol) were placed in a round-bottom flask, and potassium carbonate (943 mg, 6.82 mmol) was added and dissolved in DMF (20 mL). The mixture was reacted at 100°C for 16 h, and cooled to room temperature. Water was added at 0°C to quench the reaction. The reaction solution was extracted with EA (100 mL). The EA layer was washed with saturated saline, dried over anhydrous sodium sulfate, filtered, concentrated, and purified by silica gel column chromatography (EA:petroleum ether=1:10-1:3) to yield compound **20-23** (1.7 g, 92%) as a yellow solid.

¹H NMR (400 MHz, CDCl3) δ 7.98 (dd, *J =* 1.6, 8.0 Hz, 1H), 7.89 (s, 1H), 7.79 (d, *J =* 8.7 Hz, 1H), 7.51-7.46 (m, 1H), 7.19 (td, *J* = 1.2*,* 7.7 Hz, 1H), 7.13 (d, *J =* 2.3 Hz, 1H), 7.05 (dd, *J* = 2.3, 8.7 Hz, 1H), 6.96 (dd, *J* = 1.0, 8.4 Hz, 1H), 3.83 (s, 3H).

*m*/*z*(ESI)[M+H] ⁺=270.

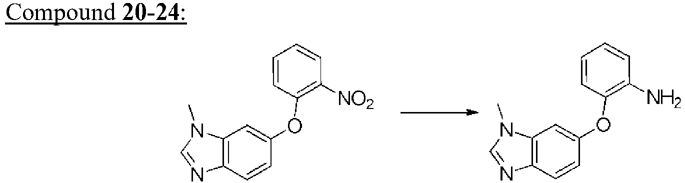

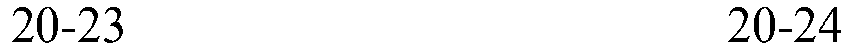

Compound **20-23** (1.7 g, 6.31 mmol) was placed in a round-bottom flask. EA (15 mL) and MeOH (10 mL) were added. Afterwards, 10% Pd/C (0.2 g) was added, and hydrogen was charged. The mixture was reacted at room temperature for 16 h. The reaction solution was filtered through diatomite, concentrated, and purified by silica gel column chromatography (methanol:CH₂Cl₂=1:30-1:10) to yield compound **20-24** (1.5 g, 99%) as a foamy solid.

¹H NMR (400 MHz, CDCl3) δ 7.83 (s, 1H), 7.75 (d, *J* = 8.7 Hz, 1H), 7.06 (dd, J=2.3, 8.8 Hz, 1H), 7.03-6.99 (m, 1H), 6.94 (d, *J* = 2.2 Hz, 1H), 6.87 (d, *J= 7.8* Hz, 2H), 6.74 (td, *J* = 1.6, 7.8 Hz, 1H), 3.88 (br s, 2H), 3.77 (s, 3H).

*m*/*z*(ESI)[M+H]⁺=240.

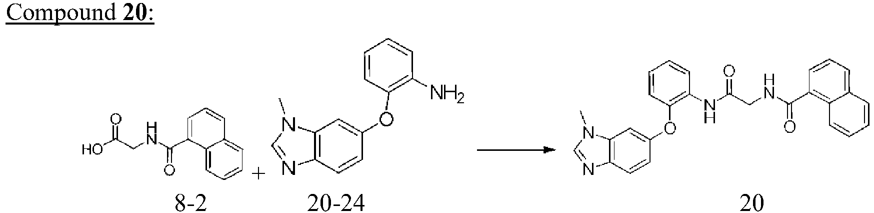

Compound **20-24** (1.55 g, 6.48 mmol) and compound **8-2** (1.78 g, 7.77 mmol) were placed in a round-bottom flask, HATU (3.7 g, 9.72 mmol) was added and dissolved in DMF (20 mL), and finally DIPEA (3.4 mL, 19.44 mmol) was added. The mixture was reacted at room temperature for 16 h. Water was added at 0°C to quench the reaction. The reaction solution was extracted with EA (100 mL). The EA layer was washed with saturated saline, dried over anhydrous sodium sulfate, filtered, concentrated, and purified by silica gel column chromatography (EA:petroleum ether=1:10-1:2) to yield a solid. The solid was recrystallized from petroleum ether and ethyl acetate to yield compound **20** (0.9 g, 31%) as a white solid.

¹H NMR (400 MHz, DMSO) δ 9.65 (s, 1H), 8.95 (d, *J =* 6.0 Hz, 1H), 8.33 (d, *J =* 8.0 Hz, 1H), 8.20 (d, *J =* 7.8 Hz, 1H), 8.18 (s, 1H), 8.02 (d, *J=* 8.2 Hz, 1H), 7.97 (d, *J =* 7.6 Hz, 1H), 7.67 (d, *J =* 8.6 Hz, 1H), 7.65 (d, *J =* 7.2 Hz, 1H), 7.57-7.46 (m, 3H), 7.31 (d, *J =* 2.2 Hz, 1H), 7.13-7.04 (m, 2H), 6.89 (dd, *J =* 2.2, 8.6 Hz, 1H), 6.81 (dd, *J =* 1.4, 7.8 Hz, 1H), 4.19 (d, *J* = 6.0 Hz, 2H), 3.77 (s, 3H).

m/z(ESI) [M+H]⁺=451.

### Preparation Example 6. Preparation of Compound 21 (MTB-1963):

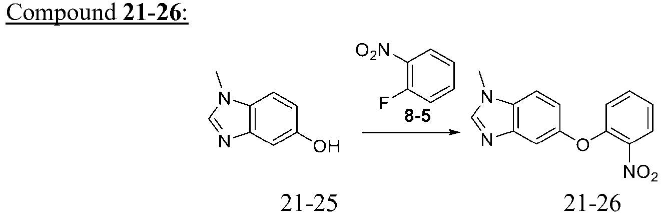

Compound **21-25** (5.0 g, 33.75 mmol) and compound **8-5** (4.33 g, 30.68 mmol) were placed in a round-bottom flask, and potassium carbonate (4.66 g, 33.75 mmol) was added and dissolved in DMF (80 mL). The mixture was reacted overnight at 100°C, and cooled to room temperature. Water was added at 0°C to quench the reaction. The reaction solution was extracted with EA (100 mL). The EA layer was washed with saturated saline, dried over anhydrous sodium sulfate, filtered, concentrated, and purified by silica gel column chromatography (EA:petroleum ether=1:10-1:2) to yield compound **21-26** (5.2 g, 57%) as a yellowish white solid.

¹H NMR (400 MHz, CDCl3) δ 7.97 (dd, *J* = 1.6, 8.0 Hz, 1H), 7.90 (s, 1H), 7.50 (d, *J=* 2.2 Hz, 1H), 7.48-7.41 (m, 1H), 7.40 (d, *J=* 8.6 Hz, 1H), 7.18 (d, *J=* 7.6 Hz, 1H), 7.13 (dd, *J =* 1.8, 8.7 Hz, 1H), 6.96 (d, *J=* 8.4 Hz, 1H), 3.90 (s, 3H).

*m*/*z*(ESI)[M+H]⁺=270.

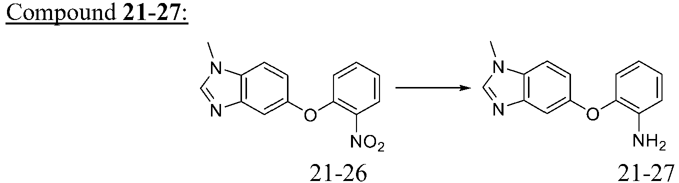

Compound **21-26** (5.2 g, 19.31 mmol) was placed in a round-bottom flask. EA (45 mL) and MeOH (30 mL) were added. Afterwards, 10% Pd/C (0.2 g) was added, and hydrogen was charged. The mixture was reacted at room temperature for 16 h. The reaction solution was filtered through diatomite, concentrated, and vacuum dried to yield compound **21-27** (4.6 g, 99%) as a foamy solid.

¹H NMR (400 MHz, CDCl3) δ 7.87 (s, 1H), 7.42 (d, *J =* 2.2 Hz, 1H), 7.34 (d, *J =* 8.6 Hz, 1H), 7.10 (dd, J=2.2, 8.6 Hz, 1H), 6.97 (td, *J* = 1.4, 7.6 Hz, 1H), 6.86-6.81 (m, 2H), 6.68-6.72 (m, 1H), 3.90 (br s, 2H), 3.86 (s, 3H).

*m*/*z*(ESI)[M+H] ⁺=240.

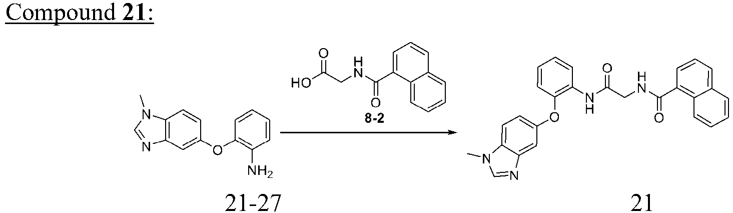

Compound **21-27** (2.52 g, 10.53 mmol) and compound **8-2** (2.9 g, 12.64 mmol) were placed in a round-bottom flask, and HATU (6.0 g, 15.8 mmol) was added and dissolved in DMF (30 mL). Finally, DIPEA (5.5 mL, 31.59 mmol) was added. The reaction solution was reacted at room temperature for 16 h. Water was added at 0°C to quench the reaction. The reaction solution was extracted with EA (100 mL). The EA layer was washed with saturated saline, dried over anhydrous sodium sulfate, filtered, concentrated, and purified by silica gel column chromatography (EA:petroleum ether=1:10-1:2) to yield a solid. The solid was recrystallized from ethyl acetate to yield compound **21** (180 mg, 21%) as a white solid.

¹H NMR (400 MHz, DMSO) δ 9.65 (s, 1H), 8.94 (t, *J* = 6.0 Hz, 1H), 8.34 (d, *J* = 8.0 Hz, 1H), 8.22 (s, 1H), 8.20 (d, *J* = 7.8 Hz, 1H), 8.02 (d, *J* = 8.2 Hz, 1H), 7.98 (d, *J* = 7.6 Hz, 1H),7.47-7.65 (m, 5H), 7.32 (d, *J =* 2.2 Hz, 1H), 7.12-7.02 (m, 3H), 6.75 (d, *J =* 7.8 Hz, 1H), 4.21 (d, *J =* 6.0 Hz, 2H), 3.85 (s, 3H).

*m*/*z*(ESI)[M+H]⁺=451.

### Preparation Example 7. Preparation of Compound 23 (MTB-1957)

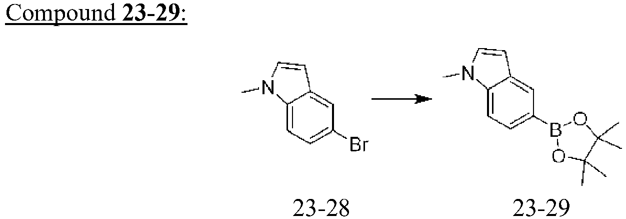

Compound **23-28** (5 g, 23.8 mmol), bis(pinacolato)diboron (9.1 g, 35.7 mmol), potassium acetate (7.0 g, 71.4 mmol), and Pd(dppf)Cl₂ (871 mg, 1.19 mmol) were added to dioxane (60 mL), reacted at 100°C for 6 h under nitrogen protection, and cooled to room temperature. EA (100 mL) and water (20 mL) were added. The reaction solution was filtered through diatomite. The filtrate was extracted. The organic phase was dried over anhydrous sodium sulfate, filtered, concentrated, and purified by silica gel column chromatography (EA:petroleum ether=1:10-1:3) to yield compound **23-29** (5.9 g, 96%) as a white solid.

¹H NMR (400 MHz, CDCl₃) δ 8.16 (s, 1H), 7.67 (d, *J =* 8.3 Hz, 1H), 7.32 (d, *J =* 8.3 Hz, 1H), 7.04 (d, *J=* 3.1 Hz, 1H), 6.50 (d, *J=* 3.1 Hz, 1H), 3.79 (s, 3H), 1.37 (s, 12H).

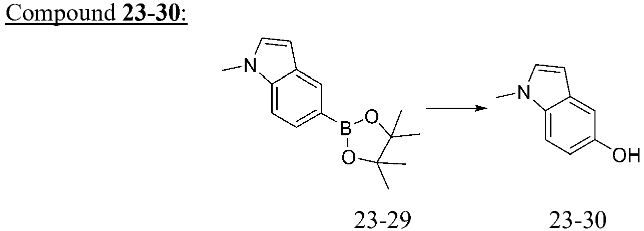

Compound **23-29** (5.9 g, 22.94 mmol) was dissolved in THF (50mL). NaHCO₃ (3.8 g, 45.88 mmol) was added. Thereafter, H₂O₂ (30%, 4.7 mL, 45.88 mmol) was added dropwise at 0°C. The reaction solution was reacted at room temperature for 3 h. A saturated sodium bisulfite solution was added to quench the reaction. The reaction solution was extracted with EA (100 mL). The organic phase was dried over anhydrous sodium sulfate, filtered, concentrated, and purified by silica gel column chromatography (methanol:CH₂Cb=1:30-1:20) to yield compound **23-30** (1.38 g, 41%) as a yellow solid.

¹H NMR (400 MHz, CDCl₃) δ 7.17 (d, *J* = 8.7 Hz, 1H), 7.08 - 6.94 (m, 2H), 6.81 (dd, *J* = 8.7, 2.4 Hz, 1H), 6.35 (dd, *J =* 3.0, 0.7 Hz, 1H), 5.01 (s, 1H), 3.75 (s, 3H).

*m*/*z*(ESI)[M+H]⁺=148.1.

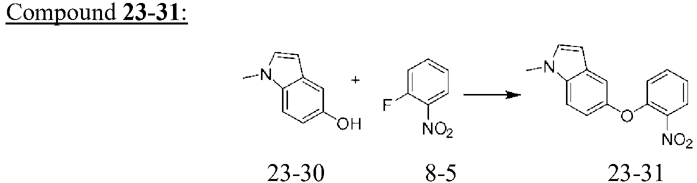

Compound **8-5** (1.2 g, 8.52 mmol), compound **23-30** (1.38 g, 9.37 mmol), and K₂CO₃ (1.3 g, 9.37 mmol) were added to DMF (30 mL), reacted at 110°C for 16 h under nitrogen protection, and cooled to room temperature. The reaction solution was extracted with EA (100 mL). The organic phase was dried over anhydrous sodium sulfate, filtered, concentrated, and purified by silica gel column chromatography (EA:petroleum ether=1:10-1:3) to yield compound **23-31**(2.08 g, 91%) as a yellow solid.

m/z(ESI)[M+H]⁺=269.1.

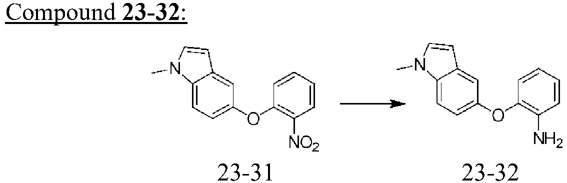

Compound **23-31** (2.08 g, 7.7 mmol) as obtained above was dissolved in a mixed solvent of EA (30 mL) and MeOH (30mL), 10% Pd/C (0.2 g) was added, and hydrogen was charged. The mixture was reacted at room temperature for 16 h. The reaction solution was filtered through diatomite, and washed with EA. The filtrate was concentrated to yield compound **23-32** (1.8 g, 98%) as a grey oily product.

*m*/*z*(ESI)[M+H]⁺=239.1.

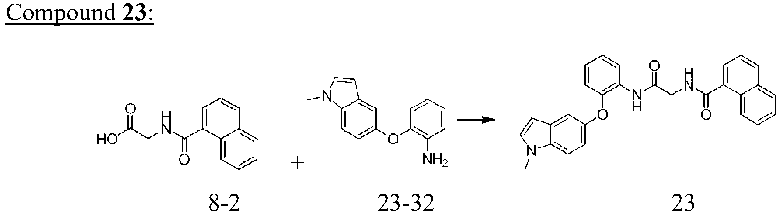

As obtained above, compound **23-32** (1.8 g, 7.55 mmol), compound **8-2** (2.1 g, 9.06 mmol), and HATU (4.3 g, 11.33 mmol) were added to DMF (25 mL), and then DIPEA (3.9 mL, 22.65 mmol) was added dropwise, and reacted at room temperature for 16 h under nitrogen protection. The reaction solution was extracted with EA (100 mL). The organic phase was dried over anhydrous sodium sulfate, filtered, concentrated, purified by silica gel column chromatography (EA:petroleum ether=1:10-1:2), and recrystallized (EA/petroleum ether) to yield compound **23** (1.7 g, 50%) as a white solid.

¹H NMR (400 MHz, CDCl₃) δ 8.54 (s, 1H), 8.42 (d, *J* = 7.1 Hz, 1H), 8.35 (d, *J=* 8.3 Hz, 1H), 7.90 (d, *J =* 8.3 Hz, 1H), 7.86 - 7.81 (m, 1H), 7.64 (dd, *J =* 7.0, 1.0 Hz, 1H), 7.54 - 7.41 (m, 2H), 7.40 - 7.32 (m, 1H), 7.30 - 7.24 (m, 2H), 7.10 (d, *J=* 3.1 Hz, 1H), 7.05 (td, *J=* 7.9, 1.3 Hz, 1H), 7.02 - 6.93 (m, 2H), 6.83 (s, 1H), 6.77 (dd, *J* = 8.1, 1.1 Hz, 1H), 6.43 (d, *J* = 3.0 Hz, 1H), 4.39 (d, *J* = 5.4 Hz, 2H), 3.80 (s, 3H).

*m*/*z*(ESI)[M+H]⁺=450.1.

### Preparation Example 8. Preparation of Compound 24 (MTB-1960):

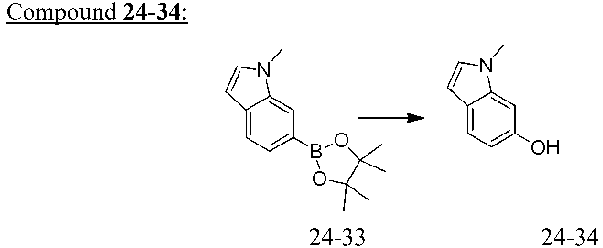

Compound **24-33** (3.4 g, 13.22 mmol) was dissolved in THF (50 mL), NaHCO₃ (2.2 g, 26.44 mmol) was added, and then H₂O₂ (30%, 2.7 mL, 26.44 mmol) was added dropwise at 0°C. The mixture was reacted at room temperature for 3 h. A saturated sodium bisulfite solution was added to quench the reaction. The reaction solution was extracted with EA (100 mL). The organic phase was dried over anhydrous sodium sulfate, filtered, concentrated, and purified by silica gel column chromatography (methanol:CH₂Cb=1:30-1:20) to yield compound **24-34** (1.9 g, 97%) as a yellow oily product.

¹H NMR (400 MHz, CDCl₃) δ 7.45 (d, *J =* 8.4 Hz, 1H), 6.93 (d, *J* = 3.1 Hz, 1H), 6.77 (d, *J =* 2.2 Hz, 1H), 6.67 (dd, *J =* 8.4, 2.2 Hz, 1H), 6.41 (dd, *J =* 3.1, 0.7 Hz, 1H), 4.82 (s, 1H), 3.70 (s, 3H).

*m*/*z*(ESI)[M+H]⁺=148.1.

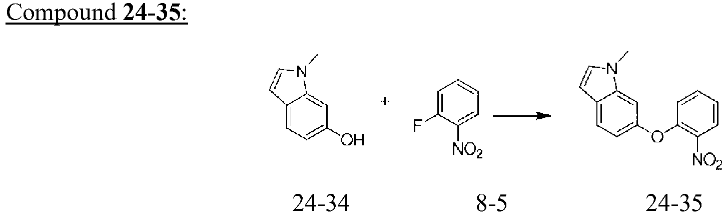

Compound **8-5** (1.5 g, 10.76 mmol), compound **24-34** (1.9 g, 12.91 mmol), and K₂CO₃ (1.6 g, 11.8 mmol) were added to DMF (30 mL), reacted at 110°C for 16 h under nitrogen protection, and cooled to room temperature. The reaction solution was extracted with EA (100 mL). The organic phase was dried over anhydrous sodium sulfate, filtered, concentrated, and purified by silica gel column chromatography (EA:petroleum ether=1:10-1:3) to yield **24-35** (2.4 g, 83%) as a yellow solid.

*m*/*z*(ESI)[M+H]⁺=269.1.

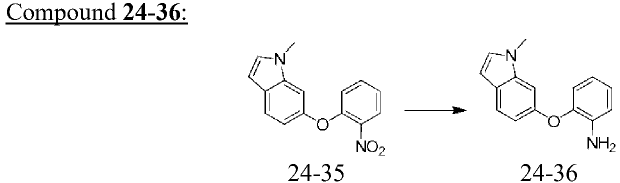

Compound **24-35** (2.4 g, 8.9 mmol) as obtained above was dissolved in a mixed solvent of EA (30 mL) and MeOH (30 mL), 10% Pd/C (0.2 g) was added, and hydrogen was charged. The mixture was reacted at room temperature for 16 h. The reaction solution was filtered and washed with EA. The filtrate was concentrated to yield compound **24-36** (2.1 g, 99%) as a grey oily product.

*m*/*z*(ESI)[M+H]⁺=239.1.

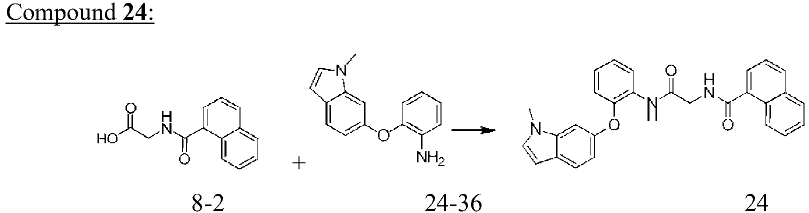

Compound **24-36** (2.1 g, 8.81 mmol), compound **8-2** (2.4 g, 10.57 mmol), and HATU (5.0 g, 13.22 mmol) were added to DMF (25 mL), and then DIPEA (4.6 mL, 26.43 mmol) was added dropwise, and reacted at room temperature for 16 h under nitrogen protection. The reaction solution was extracted with EA (100 mL). The organic phase was dried over anhydrous sodium sulfate, filtered, concentrated, purified by silica gel column chromatography (EA:petroleum ether=1:10-1:2), and recrystallized (EA/petroleum ether) to yield compound **24** (154 mg, 39%) as a white solid.

¹H NMR (400 MHz, DMSO) δ 9.66 (s, 1H), 8.98 (t, *J =* 5.9 Hz, 1H), 8.39 - 8.32 (m, 1H), 8.23 (d, *J =* 7.6 Hz, 1H), 8.06 - 7.91 (m, 2H), 7.66 (dd, *J =* 7.0, 0.9 Hz, 1H), 7.62 - 7.43 (m, 4H), 7.32 (d, *J* = 3.1 Hz, 1H), 7.20 (d, *J* = 1.9 Hz, 1H), 7.14 - 6.98 (m, 2H), 6.83 (dd, *J* = 8.5, 2.1 Hz, 1H), 6.78 (dd, *J* = 8.0, 1.5 Hz, 1H), 6.46 (d, *J* = 3.1 Hz, 1H), 4.24 (d, *J* = 6.0 Hz, 2H), 3.72 (s, 3H).

*m*/*z*(ESI)[M+H]⁺=450.1.

### Preparation Example 9. Preparation of Compound 44

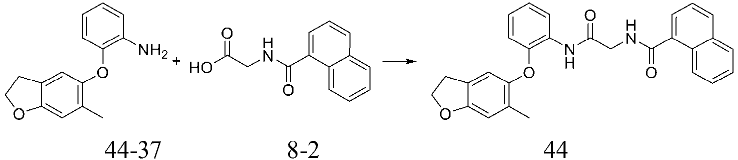

Compound **44-37** (764 mg, 3.17 mmol), compound **8-2** (872 mg, 3.80 mmol), and HATU (1.8 g, 4.76 mmol) were added to DMF (15 mL), and then DIPEA (1.7 mL, 9.51 mmol) was added dropwise, and reacted at room temperature for 16 h under nitrogen protection. The reaction solution was extracted with EA (100 mL), dried over anhydrous sodium sulfate, filtered, concentrated, purified by silica gel column chromatography (EA:petroleum ether=1:10-1:2), and recrystallized (EA/petroleum ether) to yield compound **44** (440 mg, 31%) as a yellow solid.

¹H NMR (400 MHz, CDCl₃) δ 8.68 (s, 1H), 8.38 (d, *J =* 7.5 Hz, 1H), 8.33 (d, *J =* 8.4 Hz, 1H), 7.90 (d, *J =* 8.1 Hz, 1H), 7.83 (d, *J* = 8.0 Hz, 1H), 7.64 (d, *J =* 6.8 Hz, 1H), 7.48 (t, *J =* 7.3 Hz, 1H), 7.42-7.31 (m, 2H), 7.05-6.89 (m, 3H), 6.79 (s, 1H), 6.65 (s, 1H), 6.55 (d, *J =* 7.7 Hz, 1H), 4.57 (t, *J* = 8.5 Hz, 2H), 4.41 (d, *J* = 5.0 Hz, 2H), 3.13 (t, *J* = 8.3 Hz, 2H), 2.07 (s, 3H). m/z(ESI) [M+H]⁺=453.1.

### Preparation Example 10. Preparation of Compound 90:

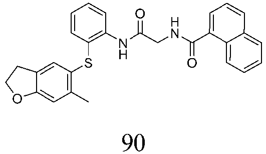

Compound **90** was prepared by the method for preparing compound **44.**

*m*/*z*(ESI) [M+H]⁺=469.1.

### Preparation Example 11. Preparation of Compound 93 (MTB-1993):

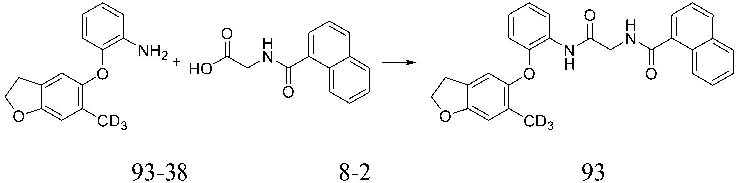

### Compound 93 was prepared from compound 93-38 by the method for preparing compound 44.

¹H NMR (400 MHz, CDCl₃) δ 8.61 (s, 1H), 8.40 (d, *J* = 7.5 Hz, 1H), 8.35 (d, *J* = 8.5 Hz, 1H), 7.92 (d, *J* = 8.2 Hz, 1H), 7.85 (d, *J* = 8.1 Hz, 1H), 7.67 (d, *J* = 6.8 Hz, 1H), 7.49 (t, *J* = 7.2 Hz, 1H), 7.40 (dd, *J* = 16.1, 8.6 Hz, 2H), 7.01 (t, *J* = 7.2 Hz, 1H), 6.95 (t, *J=* 7.2 Hz, 1H), 6.87 (t, *J* = 5.1 Hz, 1H), 6.80 (s, 1H), 6.66 (s, 1H), 6.56 (d, *J* = 7.7 Hz, 1H), 4.58 (t, *J* = 8.6 Hz, 2H), 4.43 (d, *J* = 5.4 Hz, 2H), 3.15 (t, *J* = 8.6 Hz, 2H). m/z(ESI) [M+H]⁺=456.3.

m/z(ESI) [M+H]⁺=457.2.

### Preparation Example 12. Preparation of Compound 99

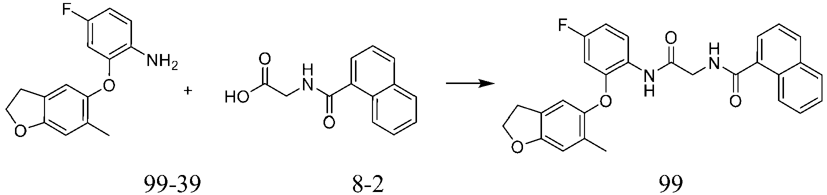

Compound **99-39** (500 mg, 1.93 mmol), **8-2** (575 mg, 2.51 mmol), and HATU (1.09 g, 2.90 mmol) were added to DMF (10 mL), and then DIPEA (0.98 mL, 5.79 mmol) was added dropwise, and reacted overnight at room temperature under nitrogen protection. The reaction solution was extracted with ethyl acetate, dried over anhydrous sodium sulfate, filtered, and concentrated. The residue was subjected to silica gel column chromatography (EA:petroleum ether=1:10-1:2) and then recrystallization (EA/petroleum ether) to yield compound **99** (408 mg, 45%) as an off-white solid. ¹H NMR (400 MHz, CDCl₃) δ 8.56 (s, 1H), 8.36-8.30 (m, 2H), 7.92 (d, *J* = 8.3 Hz, 1H), 7.85 (d, *J* = 8.2 Hz, 1H), 7.67 (dd, *J* = 7.0, 1.0Hz, 1H), 7.49 (td, *J* = 7.0, 1.0 Hz, 1H), 7.43 - 7.34 (m, 2H), 6.86 (t, *J* = 5.0 Hz, 1H), 6.82 (s, 1H), 6.70 (td, *J* = 8.5, 2.7 Hz,1H), 6.67 (s, 1H), 6.28 (dd, *J=* 9.8, 2.7 Hz, 1H), 4.60 (t, *J* = 8.7 Hz, 2H), 4.43 (d, *J=* 5.5 Hz, 2H), 3.17 (t, *J* = 8.6 Hz, 2H), 2.07 (s, 3H). m/z(ESI) [M+H]⁺=471.3.

### Preparation Example 13. Preparation of Compound 100 (MTB-2006)

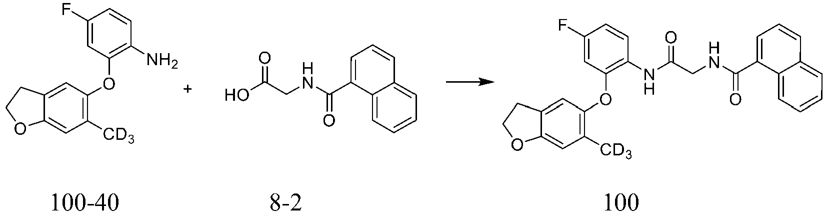

Compound **100-40** (675 mg, 2.57 mmol), **8-2** (766 mg, 3.34 mmol), and HATU (1.46 g, 3.86 mmol) were added to DMF (10 mL), and then DIPEA (1.3 mL, 7.71 mmol) was added dropwise, and reacted overnight at room temperature under nitrogen protection. The reaction solution was extracted with ethyl acetate, dried over anhydrous sodium sulfate, filtered, and concentrated. The residue was purified by silica gel column chromatography (EA:petroleum ether=1:10-1:2) and then recrystallized (EA/petroleum ether) to yield compound **100** (450 mg, 37%) as an off-white solid.

¹H NMR (400 MHz, CDCl₃) δ 8.59 (s, 1H), 8.39 - 8.25 (m, 2H), 7.91 (d, *J =* 8.3 Hz, 1H), 7.85 (d, *J =* 8.2 Hz, 1H), 7.66 (dd, *J =* 7.1, 1.0 Hz, 1H), 7.52 - 7.45 (m, 1H), 7.42 - 7.33 (m, 2H), 6.91 (t, *J =* 5.2 Hz, 1H), 6.81 (s, 1H), 6.72 - 6.67 (m, 1H), 6.66 (s, 1H), 6.27 (dd, *J =* 9.8, 2.7 Hz, 1H), 4.59 (t, *J* = 8.7 Hz, 2H), 4.42 (d, *J =* 5.5 Hz, 2H), 3.16 (t, *J =* 8.7 Hz, 2H). *m*/*z*(ESI) [M+H]⁺=474.4.

### Preparation Example 14. Preparation of Other Analogs

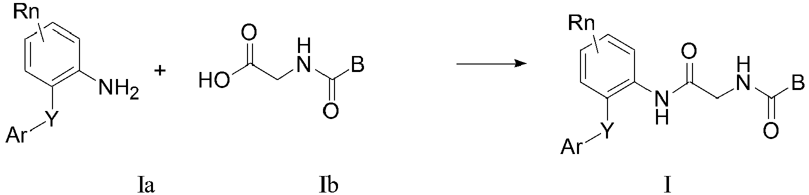

The target products shown in Table 1 were prepared by condensation of compounds Ia and Ib using similar methods.

**Table 1**

| No. | Structure of Compound | *m*/*z* | No. | | *m*/*z* |
|---|---|---|---|---|---|
| 1 | 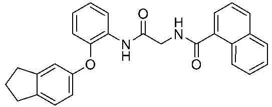 | 437 | 2 | 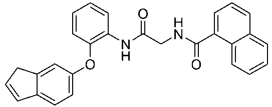 | 435 |
| 3 | 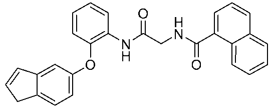 | 435 | 4 | 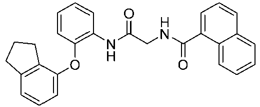 | 437 |
| 5 | 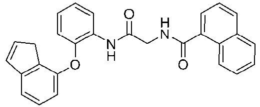 | 435 | 6 | 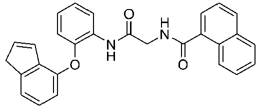 | 435 |
| 7 | 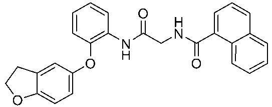 | 439 | 10 | 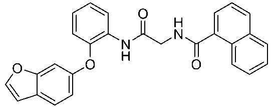 | 437 |
| 11 | 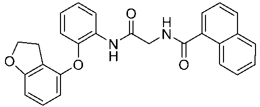 | 439 | 12 | 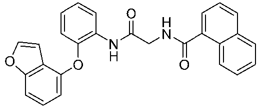 | 437 |
| 13 | 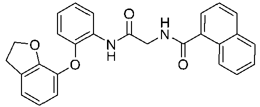 | 439 | 14 | 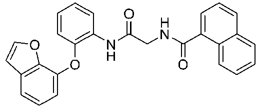 | 437 |
| 16 | 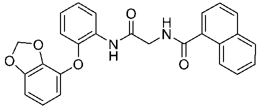 | 441 | 18 | 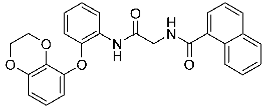 | 455 |
| 19 | 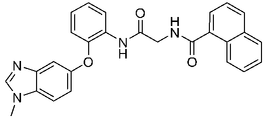 | 451 | 22 | 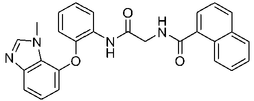 | 451 |
| 25 | 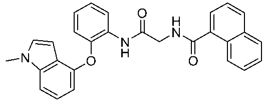 | 450 | 26 | 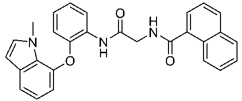 | 450 |
| 27 | 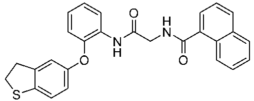 | 455 | 28 | 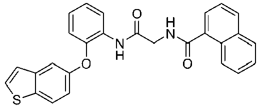 | 453 |
| 29 | 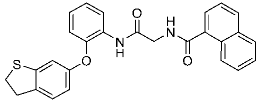 | 455 | 30 | 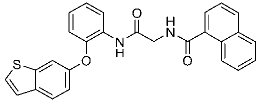 | 453 |
| 31 | 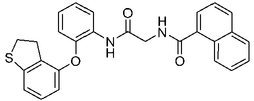 | 455 | 32 | 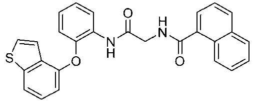 | 453 |
| 33 | 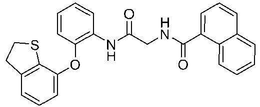 | 455 | 34 | 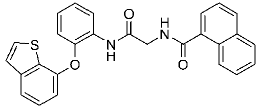 | 453 |
| 35 | 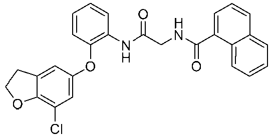 | 473 | 36 | 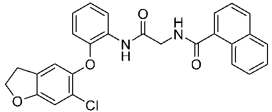 | 473 |
| 37 | 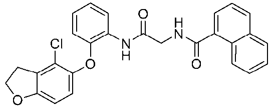 | 473 | 38 | 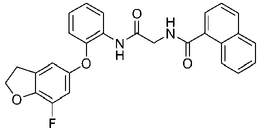 | 457 |
| 39 | 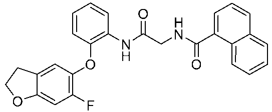 | 457 | 40 | 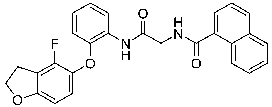 | 457 |
| 41 | 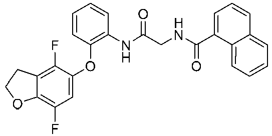 | 475 | 42 | 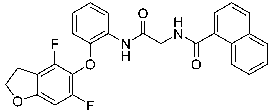 | 475 |
| 43 | 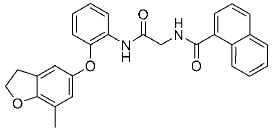 | 453 | 45 | 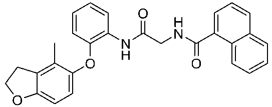 | 453 |
| 46 | 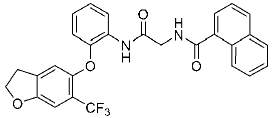 | 507 | 47 | 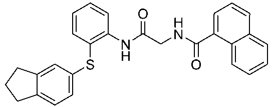 | 453 |
| 48 | 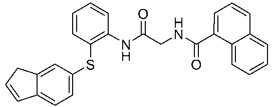 | 451 | 49 | 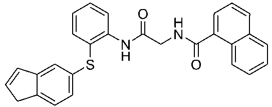 | 451 |
| 50 | 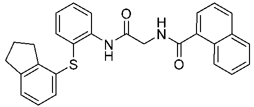 | 453 | 51 | 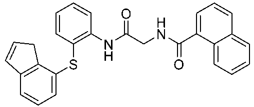 | 451 |
| 52 | 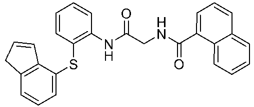 | 451 | 53 | 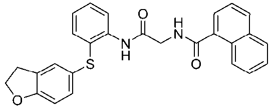 | 455 |
| 54 | 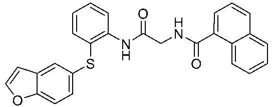 | 453 | 55 | 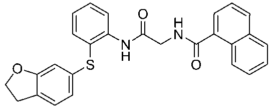 | 455 |
| 56 | 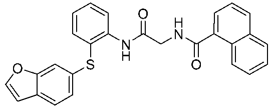 | 453 | 57 | 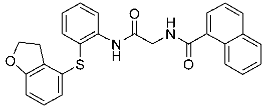 | 455 |
| 58 | 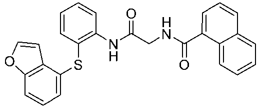 | 453 | 59 | 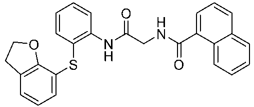 | 455 |
| 60 | 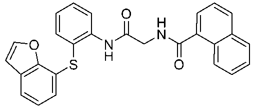 | 453 | 61 | 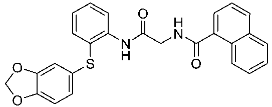 | 457 |
| 62 | 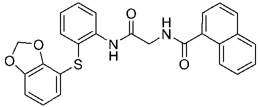 | 457 | 63 | 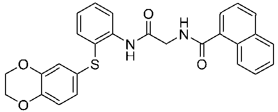 | 471 |
| 64 | 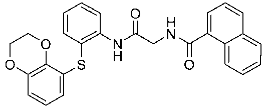 | 471 | 65 | 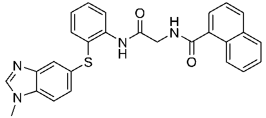 | 467 |
| 66 | 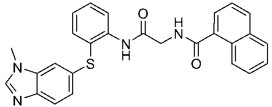 | 467 | 67 | 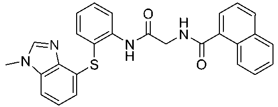 | 467 |
| 68 | 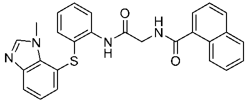 | 467 | 69 | 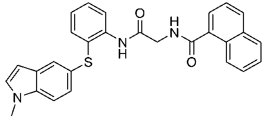 | 466 |
| 70 | 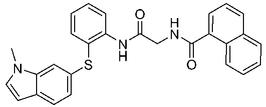 | 466 | 71 | 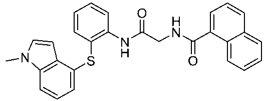 | 466 |
| 72 | 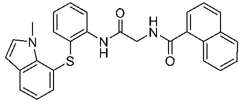 | 466 | 73 | 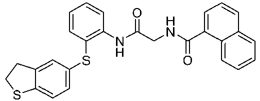 | 471 |
| 74 | 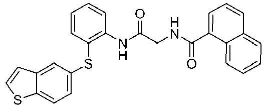 | 469 | 75 | 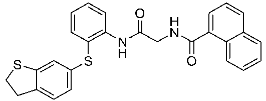 | 471 |
| 76 | 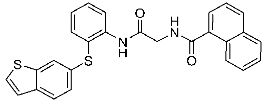 | 469 | 77 | 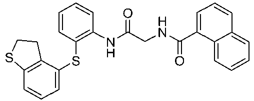 | 471 |
| 78 | 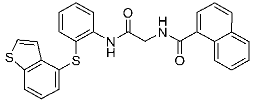 | 469 | 79 | 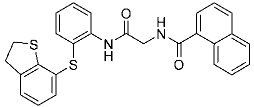 | 471 |
| 80 | 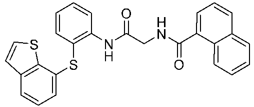 | 469 | 81 | 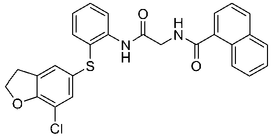 | 489 |
| 82 | 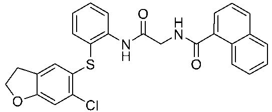 | 489 | 83 | 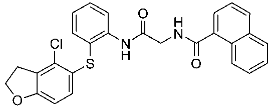 | 489 |
| 84 | 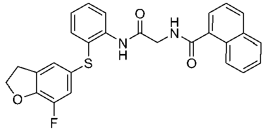 | 473 | 85 | 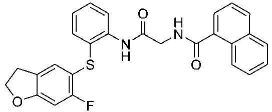 | 473 |
| 86 | 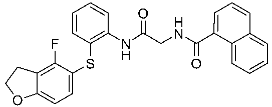 | 473 | 87 | 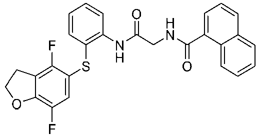 | 491 |
| 88 | 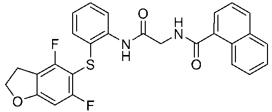 | 491 | 89 | 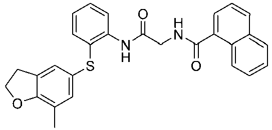 | 469 |
| 91 | 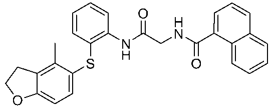 | 469 | 92 | 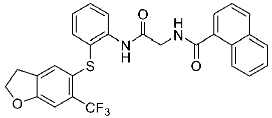 | 523 |
| 94 | 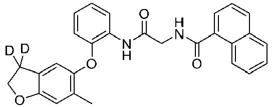 | 455 | 95 | 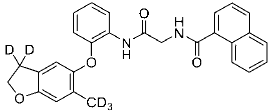 | 458 |
| 96 | 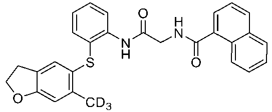 | 472 | 97 | 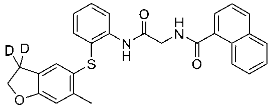 | 471 |
| 98 | 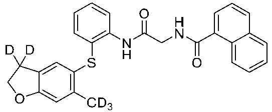 | 474 | 101 | 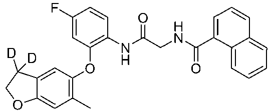 | 473 |
| 102 | 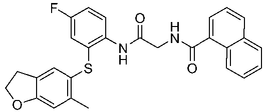 | 487 | 103 | 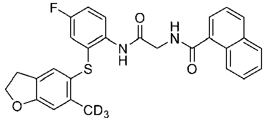 | 490 |
| 104 | 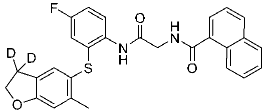 | 489 | | | |

### Preparation Example 15. Preparation of Comparative Compound 1 (MTB-1962)

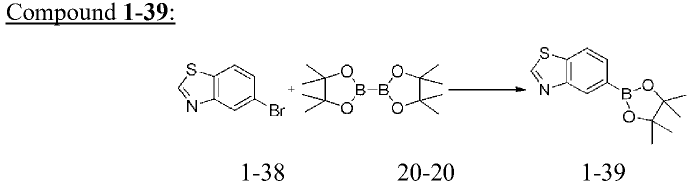

Compound **1-38** (15.0 g, 70.1 mmol) and compound **20-20** (21.35 g, 84.1 mmol) were placed in a round-bottom flask, and potassium acetate (20.63 g, 210.2 mmol) and PdCl₂(dppf) (2.56 g, 3.5 mmol), and dissolved in 1,4-dioxane (140 mL). The mixture was reacted at 90°C for 5 h. Water was added at 0°C to quench the reaction. The reaction solution was extracted with EA (100 mL), and filtered through diatomite. The organic phase was washed two times with saturated saline, dried over anhydrous sodium sulfate, filtered, concentrated, and purified by silica gel column chromatography (EA:petroleum ether=1:10-1:3) to yield compound **1-39** (18.3 g, 99%) as a yellowish white solid.

*m*/*z*(ESI)[M+H]⁺=262.

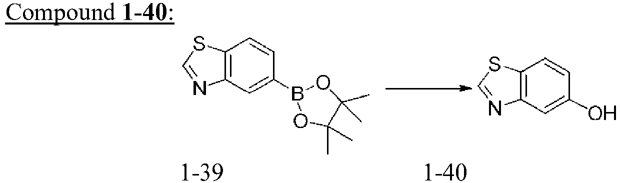

Compound **1-39** (6.0 g, 22.98 mmol) was placed in a round-bottom flask, and dissolved in THF (50 mL). Sodium bicarbonate (3.86 g, 45.96 mmol) was added. Hydrogen peroxide (30%, 4.7 mL, 45.96 mmol) was added dropwise at 0°C. The mixture was reacted with stirring at room temperature for 3 h. Saturated sodium bisulfite was added to quench the reaction. The reaction solution was extracted with EA (100 mL). The EA layer was washed with water one time and with saturated saline one time, dried over anhydrous sodium sulfate, concentrated, and recrystallized from petroleum ether and ethyl acetate to yield 2.66 g of compound **1-40** as a yellowish white solid, which was directly used for the next step.

¹H NMR (400 MHz, DMSO) δ 9.73 (s, 1H), 9.30 (s, 1H), 7.92 (d, *J =* 8.6 Hz, 1H), 7.40 (d, *J =* 2.3 Hz, 1H), 6.95 (dd, *J* = 2.3, 8.6 Hz, 1H).

*m*/*z*(ESI)[M+H]⁺=152.

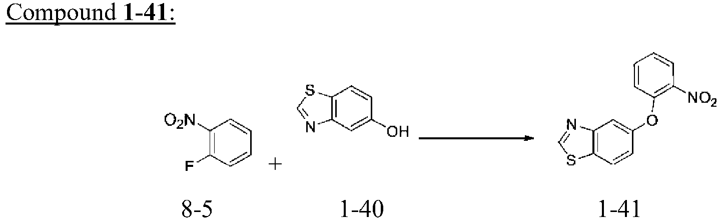

Compound **1-40** (2.66 g, 17.6 mmol) and compound **8-5** (2.26 g, 16.0 mmol) were placed in a round-bottom flask, and potassium carbonate (2.43 g, 17.6 mmol) was added and dissolved in DMF (40 mL). The mixture was reacted at 100°C for 16 h, and cooled to room temperature. Water was added at 0°C to quench the reaction. The reaction solution was extracted with EA (100 mL). The EA layer was washed with saturated saline, dried over anhydrous sodium sulfate, filtered, concentrated, and purified by silica gel column chromatography (EA:petroleum ether=1:10-1:3) to yield compound **1-41** (4.43 g, 92%) as a yellow oily product.

¹H NMR (400 MHz, CDCl3) δ 9.07 (s, 1H), 8.03 (dd, *J* = 1.7, 8.2 Hz, 1H), 7.97 (d, *J =* 8.6 Hz, 1H), 7.74 (d, *J* = 2.3 Hz, 1H), 7.58-7.54 (m, 1H), 7.30-7.26 (m, 2H), 7.12 (dd, *J* = 1.2, 8.3 Hz, 1H).

*m*/*z*(ESI)[M+H] ⁺=273.

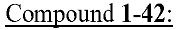

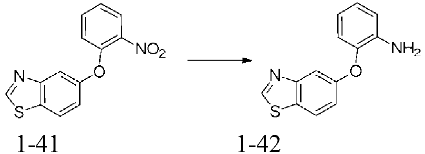

Compound **1-41** (4.43 g, 16.3 mmol) was placed in a round-bottom flask. EA (30 mL) and MeOH (20 mL) were added. Afterwards, 10% Pd/C (0.2 g) was added, and hydrogen was charged. The mixture was reacted at room temperature for 16 h. The reaction solution was filtered through diatomite, concentrated, and vacuum dried to yield compound **1-42** (4.28 g) as a yellow oily product, which was directly used for the next step.

¹H NMR (400 MHz, CDCl3) δ 9.01 (s, 1H), 7.89 (d, *J* = 8.7 Hz, 1H), 7.68 (d, *J* = 2.3 Hz, 1H), 7.23 (dd, *J =* 2.3, 8.7 Hz, 1H), 7.05 (td, *J =* 1.3, 7.8 Hz, 1H), 6.96 (dd, *J =* 1.3, 7.8 Hz, 1H), 6.87 (dd, *J* = 1.5, 7.9 Hz, 1H), 6.76 (td, *J =* 1.5, 7.8 Hz, 1H), 3.84 (br s, 2H).

*m*/*z*(ESI)[M+H] ⁺=243.

### Preparation of Compound 44-37

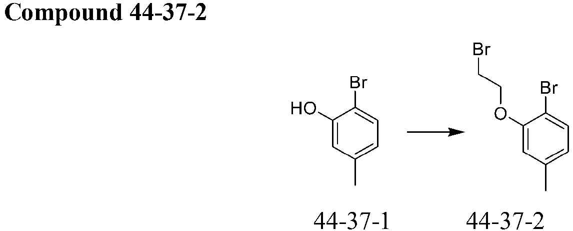

Compound **44-37-1** (10 g, 53.46 mmol), 1,2-dibromoethane (30.1 g, 160.38 mmol), and potassium carbonate (11.1 g, 80.19 mmol) were added to acetonitrile (150 mL), reacted overnight at 85°C under nitrogen protection, cooled to room temperature, filtered, concentrated, and purified by silica gel column chromatography (EA:petroleum ether=1:10-1:3) to yield compound **44-37-2** (7.4 g, 47%) as a colorless oily product.

¹H NMR (400 MHz, CDCl₃) δ 7.40 (d, *J* = 7.9 Hz, 1H), 6.72(d, *J* = 1.5 Hz, 1H), 6.69 (dd, *J* = 7.9, 1.5 Hz, 1H), 4.32 (t, *J =* 6.6 Hz, 2H), 3.67 (t, *J* = 6.6 Hz, 2H), 2.31 (s, 3H).

Compound **44-37-1** was reacted with 2-bromoethanol in the presence of potassium carbonate, and then brominated to also yield compound **44-37-2.**

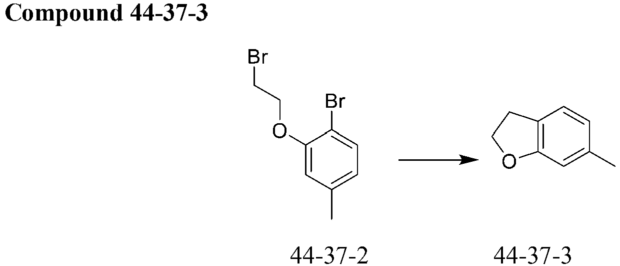

Compound **44-37-2** (7.4 g, 25.17 mmol) was dissolved in THF (50 mL), and n-BuLi (13.1 mL, 32.72 mmol, 2.5M) was added dropwise at -78°C under nitrogen protection, and reacted at -78°C for 2 h. Water was added to quench the reaction. The reaction solution was extracted with EA, concentrated, and purified by silica gel column chromatography (EA:petroleum ether=1:10-1:3) to yield compound **44-37-3** (3.06 g, 90%) as a light yellow oily product.

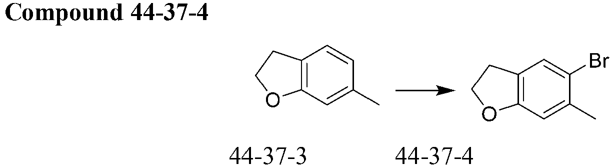

Compound **44-37-3** (3.06 g, 22.81 mmol) was dissolved in acetonitrile (45 mL), and NBS (4.46 g, 25.08 mmol) was added in batch at 0°C. Thereafter, the mixture was reacted at room temperature for 16 h, and distilled to remove the solvent. EA was added. The organic phase was washed with an aqueous saturated sodium bicarbonate solution and with saturated saline, dried over anhydrous sodium sulfate, filtered, and concentrated to yield compound **44-37-4** (4.8 g, 99%) as a light yellow solid.

¹H NMR (400 MHz, CDCl₃) δ 7.31 (s, 1H), 6.67(s, 1H), 4.55 (t, *J =* 8.7 Hz, 2H), 3.16 (t, *J* = 8.7 Hz, 2H), 2.32 (s, 3H).

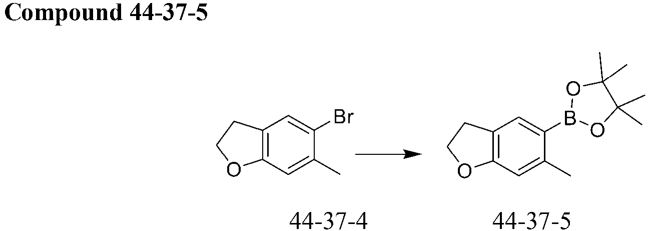

Compound **44-37-4** (4.8 g, 22.53 mmol), bis(pinacolato)diboron (6.9 g, 27.03 mmol), potassium acetate (6.6 g, 67.59 mmol), and Pd(dppf)Cl₂ (824 mg, 1.13 mmol) were added to 1,4-dioxane (50 mL), reacted at 90°C for 6 h under nitrogen protection, and cooled to room temperature. EA and water were added. The reaction solution was filtered through diatomite. The filtrate was extracted. The organic phase was dried over anhydrous sodium sulfate, filtered, concentrated, and purified by silica gel column chromatography (EA:petroleum ether=1:10-1:3) to yield compound **44-37-5** (2.7 g, 46%) as a light yellow solid.

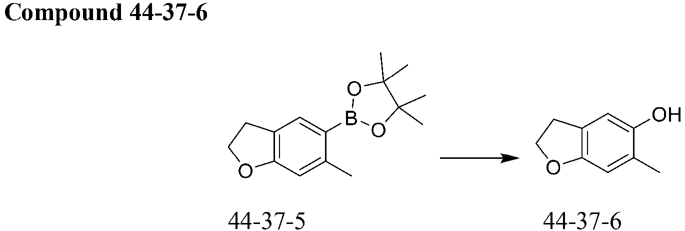

Compound **44-37-5** (2.7 g, 10.38 mmol) was dissolved in THF (20 mL). NaHCO₃ (1.7 g, 20.76 mmol) was added. Thereafter, 30% H₂O₂ (2.1 mL, 20.76 mmol) was added dropwise at 0°C. The mixture was reacted at room temperature for 3 h. A saturated sodium bisulfite solution was added to quench the reaction. The reaction solution was extracted with EA, dried over anhydrous sodium sulfate, filtered, concentrated, and purified by silica gel column chromatography (methanol:CH₂Cl₂=1:30-1:20) to yield compound **44-37-6** (600 mg, 38%) as a colorless oily product.

¹H NMR (400 MHz, CDCl₃) δ 6.66 (s, 1H), 6.56 (s, 1H), 4.51 (t, *J* = 8.7 Hz, 2H), 4.33 (s, 1H), 3.13 (t, *J =* 8.7 Hz, 2H), 2.19 (s, 3H).

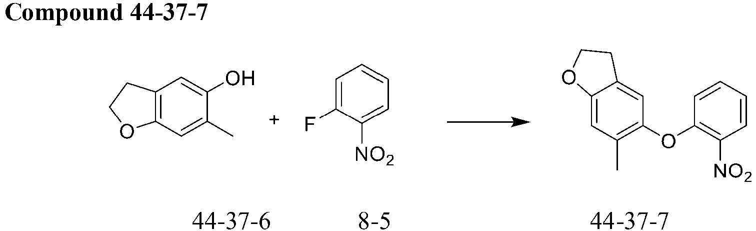

Compound **44-37-6** (600 mg, 4.0 mmol), **8-5** (470 mg, 3.33 mmol), and K₂CO₃ (506 mg, 3.66 mmol) were added to DMF (10 mL), reacted at 100°C for 16 h under nitrogen protection, and cooled to room temperature. The reaction solution was extracted with EA. The organic phase was washed with 10% NaOH solution and with saturated saline, dried over anhydrous sodium sulfate, filtered, and concentrated to yield compound **44-37-7** (860 mg, 79%) as a brown oily product.

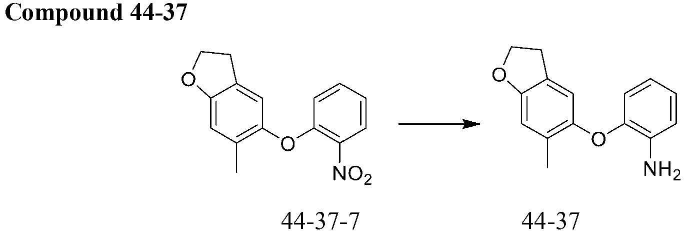

Compound **44-37-7** (860 mg, 3.17 mmol) was dissolved in a mixed solvent of ethyl acetate (10 mL) and MeOH (10 mL), and 10% Pd/C (100 mg) was added. The mixture was reacted at room temperature for 16 h under hydrogen atmosphere, filtered through diatomite, and washed with EA. The filtrate was concentrated to yield compound **44-37** (764 mg, 99%) as a brown oily product. m/z(ESI) [M+H]⁺=242.1.

### Preparation of Compound 93-38

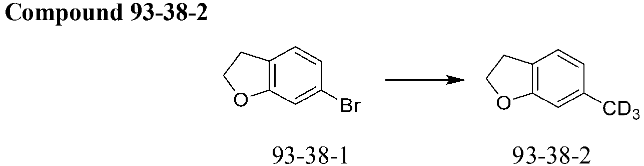

Compound **93-38-1** (10 g, 50.24 mmol) was dissolved in THF (100 mL), and n-BuLi (22 mL, 55.26 mmol, 2.5M) was added dropwise at -78°C under nitrogen protection, and stirred at -78°C for 30 min. CD₃I (8.7 g, 60.29 mmol) was dissolved in THF (20 mL) and added dropwise to the reaction system, reacted at -78°C for 1 h, and then heated to room temperature and reacted for 1 h. A saturated ammonium chloride solution was added to quench the reaction. The reaction solution was extracted with EA. The organic phase was washed with saturated saline, dried over anhydrous sodium sulfate, filtered, and concentrated to yield compound **93-38-2** (6.3 g, 91%) as a light yellow oily product.

¹H NMR (400 MHz, CDCl₃) δ 7.07 (d, *J* = 7.4 Hz, 1H), 6.66 (dd, *J* = 7.4, 1.4 Hz, 1H), 6.63 (d, *J* = 1.4 Hz, 1H), 4.55 (t, *J =* 8.7Hz, 2H), 3.16 (t, *J =* 8.7 Hz, 2H).

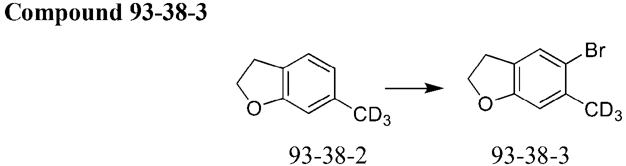

Compound **93-38-2** (6.3 g, 45.92 mmol) was weighed into a round-bottom flask. Acetonitrile (90 mL) was added. NBS (9.0 g, 50.51 mmol) was added in batch. The mixture was reacted at room temperature for 16 h. the reaction solution was distilled to remove the solvent, and extracted with EA and water. The organic layer was washed with saturated sodium bicarbonate and saturated saline in this order, dried over anhydrous sodium sulfate, filtered, and concentrated to yield compound **93-38-3** (9.9 g, 99%) as a yellow solid.

¹H NMR (400 MHz, CDCl₃) δ 7.31 (s, 1H), 6.67 (s, 1H), 4.56 (t, *J =* 8.7 Hz, 2H), 3.17 (t, *J* = 8.7 Hz, 2H).

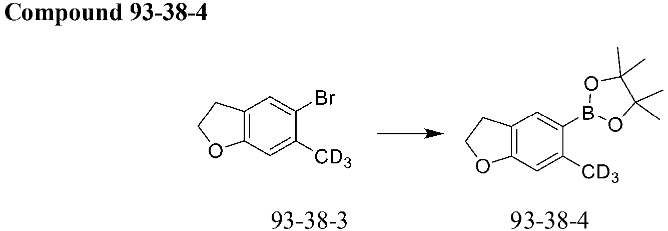

Compound **93-38-3** (9.9 g, 45.81 mmol), bis(pinacolato)diboron (17.4 g, 68.72 mmol), potassium acetate (13.5 g, 137.43 mmol), and Pd(dppf)Cl₂ (1.7 g, 29 mmol) were added to 1,4-dioxane (100 mL), reacted at 100°C for 16 h under nitrogen protection, and cooled to room temperature. EA and water were added. The reaction solution was filtered through diatomite. The filtrate was extracted. The organic phase was dried over anhydrous sodium sulfate, filtered, concentrated, and purified by silica gel column chromatography (EA:petroleum ether=1:10-1:3) to yield compound **93-38-4** (7.7 g, 64%) as a light yellow oily product.

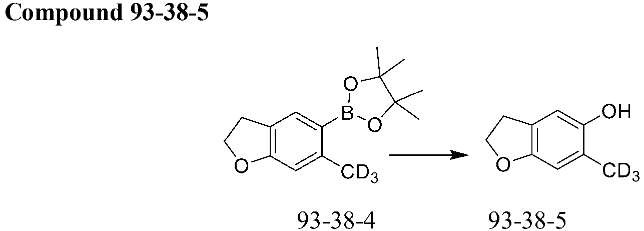

Compound **93-38-4** (7.7 g, 29.26 mmol) was dissolved in THF (70 mL), NaHCO₃ (4.9 g, 58.52 mmol) was added, and then H₂O₂ (6 mL, 58.52 mmol, 30%) was added dropwise at 0°C. The mixture was reacted at room temperature for 16 h. A saturated sodium bisulfite solution was added to quench the reaction. The reaction solution was extracted with EA, dried over anhydrous sodium sulfate, filtered, concentrated, and purified by silica gel column chromatography (methanol:CH₂Cl₂=1:30-1:20) to yield compound **93-38-5** (2.4 g, 54%) as a light yellow solid.

¹H NMR (400 MHz, CDCl₃) δ 6.66 (s, 1H), 6.56 (s, 1H), 4.51 (t, *J* = 8.6 Hz, 2H), 4.34 (s, 1H), 3.13 (t, *J* = 8.6 Hz, 2H).

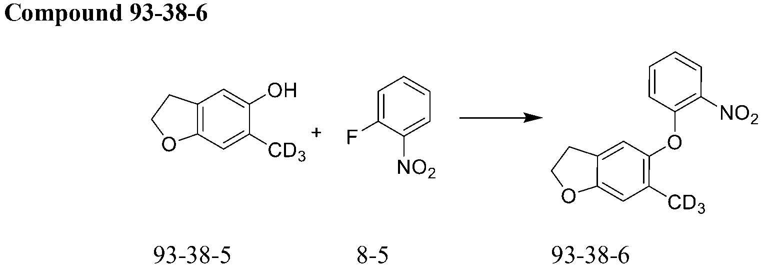

Compound **93-38-5** (2.4 g, 15.67 mmol), **8-5** (2.0 g, 14.24 mmol), and K₂CO₃ (2.2 g, 15.67 mmol) were added to DMF (30 mL), reacted at 100°C for 16 h under nitrogen protection, and cooled to room temperature. The reaction solution was extracted with EA. The organic phase was washed with 10% NaOH solution and with saturated saline, dried over anhydrous sodium sulfate, filtered, concentrated to yield compound **93-38-6** (1.9 g, 44%) as a yellow solid.

¹H NMR (400 MHz, CDCl₃) δ 7.91 (dd, *J* = 8.1, 1.7 Hz, 1H), 7.43 - 7.37 (m, 1H), 7.09 - 7.04 (m, 1H), 6.85 (s, 1H), 6.75 (dd, *J* = 8.5, 1.0 Hz, 1H), 6.68 (s, 1H), 4.59 (t, *J* = 8.6 Hz, 2H), 3.18 (t, *J* = 8.6 Hz, 2H).

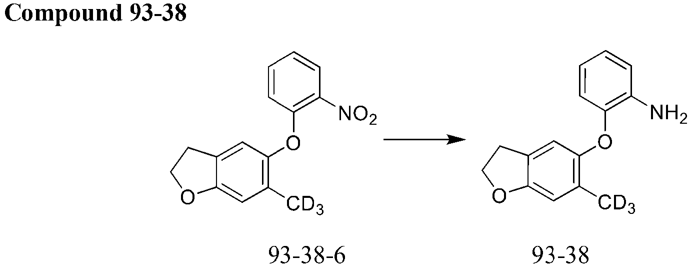

Compound **93-38-6** (1.7 g, 6.20 mmol) and NH₄Cl (2.0 g, 37.2 mmol) were added to EtOH (40 mL), and then zinc powder (2.4 g, 37.2 mmol) was added in batch. The mixture was reacted at 80°C for 16 h under nitrogen protection, and filtered. The filtrate was concentrated. The residue was purified by silica gel column chromatography (methanol:CH₂Cl₂ = 1 :30-1: 10) to yield compound **93-38** (1.51 g, 99%) as a red oily product. m/z(ESI) [M+H]⁺=245.1.

### Preparation of Compound 99-39

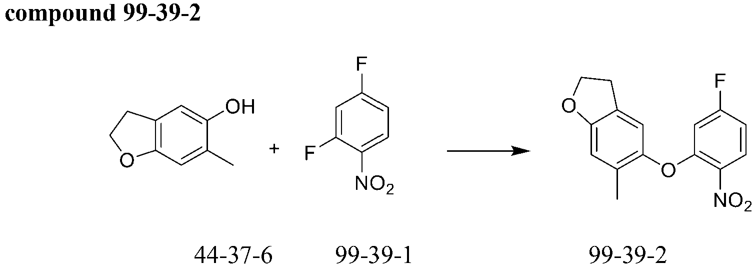

Compound **44-37-6** (500 mg, 3.33 mmol) and **99-39-1** (525 mg, 3.33 mmol) were dissolved in toluene (20 mL), and t-BuOK (385 mg, 3.43 mmol) was added in batch at 0°C. Thereafter, the mixture was reacted at room temperature for 2 h. Water was added to quench the reaction. The reaction solution was extracted with EA. The organic phase was washed with saturated saline, dried over anhydrous sodium sulfate, filtered, concentrated, and purified by silica gel column chromatography (EA:petroleum ether=1:10-1:3) to yield compound **99-39-2** (320 mg, 33%) as a yellow solid. *m*/*z*(ESI) [M+H]⁺=290.1.

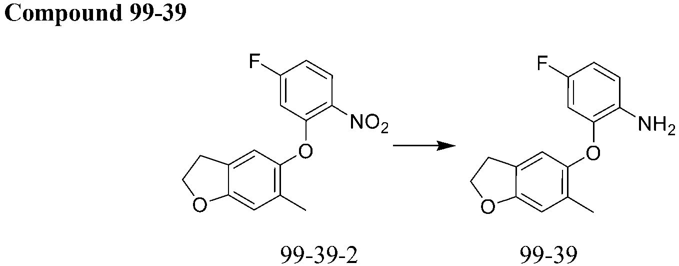

Compound **99-39-2** (500 mg, 1.73 mmol) and NH₄Cl (555 mg,10.38 mmol) were added to THF (20 mL) and water (10 mL), and then zinc powder (673 mg, 10.38 mmol) was added in batch. The mixture was reacted at 70°C for 5 h under nitrogen protection, and filtered. The filtrate was extracted with EA. The organic phase was dried over anhydrous sodium sulfate, filtered, and concentrated to obtain compound **99-39** (325 mg, 72%) as a brown oily product. *m*/*z*(ESI) [M+H]⁺=260.1.

### Preparation of Compound 100-40

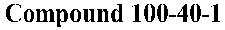

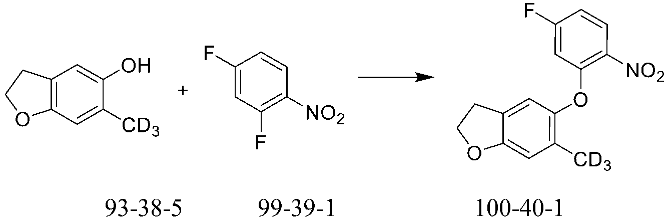

Compound **93-38-5** (1.0 g, 6.53 mmol) and compound **99-39-1** (1.0 g, 6.53 mmol) were dissolved in toluene (20 mL), and t-BuOK (769 mg, 6.86 mmol) was added in batch at 0°C. Thereafter, the mixture was reacted at room temperature for 2 h. Water was added to quench the reaction. The reaction solution was extracted with EA. The organic phase was washed with saturated saline, dried over anhydrous sodium sulfate, filtered, concentrated, and purified by silica gel column chromatography (EA:petroleum ether=1:10-1:3) to yield compound **100-40-1** (750 mg, 39%) as a yellow solid (determining the nitro group-directed ortho-selective substitution reaction according to the literature).

¹H NMR (400 MHz, CDCl₃) δ 8.02 (dd, *J=* 9.1, 5.8 Hz, 1H), 6.87 (s, 1H), 6.78 - 6.73 (m, 1H), 6.69 (s, 1H), 6.43 (dd, *J* = 10.1, 2.6 Hz, 1H), 4.60 (t, *J* = 8.7 Hz, 2H), 3.20 (t, *J* = 8.7 Hz, 2H).

¹³C NMR (101 MHz, CDCl₃) δ 165.61 (d, *J* = 256.6 Hz), 158.17, 154.77 (d, *J* = 10.8 Hz), 145.11, 135.86, 129.89, 128.18 (d, *J* = 11.1 Hz), 126.43, 117.82, 111.72, 108.54 (d, *J* = 23.9 Hz), 104.20 (d, *J* = 27.3 Hz), 71.77, 29.70, 15.57 - 14.99 (m).

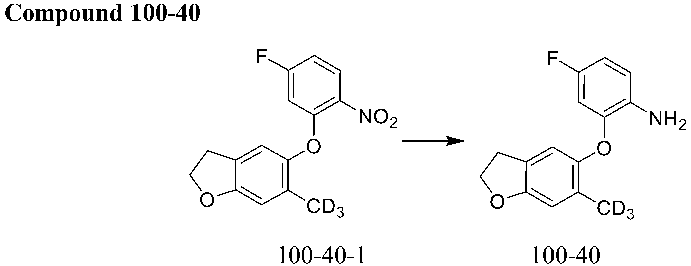

Compound **100-40-1** (750 mg, 2.57 mmol) and NH₄Cl (825 mg, 15.42 mmol) were added to THF (20 mL) and water (10 mL), and then zinc powder (1.0 g, 15.42 mmol) was added in batch. The mixture was reacted at 70°C for 5 h under nitrogen protection, and filtered. The filtrate was extracted with EA. The organic phase was dried over anhydrous sodium sulfate, filtered, and concentrated to yield compound **100-40** (670 mg, 99%) as a brown oily product. m/z(ESI) [M+H]⁺=263.1.

### Comparative Compound 1:

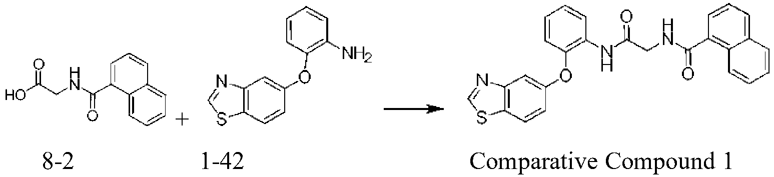

Compound **1-42** (2.0 g, 8.25 mmol) and compound **8-2** (2.27 g, 9.9 mmol) were placed in a round-bottom flask, and HATU (4.7 g, 12.38 mmol) was added and dissolved in DMF (25 mL). Finally, DIPEA (4.3 mL, 24.75 mmol) was added. The mixture was reacted at room temperature for 16 h. Water was added at 0°C to quench the reaction. The reaction solution was extracted with EA (100 mL). The EA layer was washed with saturated saline, dried over anhydrous sodium sulfate, filtered, concentrated, and purified by silica gel column chromatography (EA:petroleum ether=1:10-1:2) to yield a solid. The solid was recrystallized from petroleum ether and ethyl acetate to yield **comparative compound 1** (1.48 g, 40%) as a white solid.

¹H NMR (400 MHz, CDCl3) δ 9.04 (s, 1H), 8.62 (s, 1H), 8.50 (d, *J =* 8.2 Hz, 1H), 8.34 (d, *J* = 8.0 Hz, 1H), 7.93 (d, *J =* 8.2 Hz, 1H), 7.88-7.84 (m, 2H), 7.72 (d, *J* = 2.3 Hz, 1H), 7.60 (d, *J* = 7.0 Hz, 1H), 7.53-7.45 (m, 2H), 7.38 (t, *J* = 7.8 Hz, 1H), 7.22-7.18 (m, 2H), 7.09 (t, *J= 7.8* Hz, 1H), 6.95 (d, *J* = 8.2 Hz, 1H), 6.82 (t, *J=* 5.0 Hz, 1H), 4.38 (d, *J* = 5.5 Hz, 2H).

*m*/*z*(ESI)[M+H]⁺=454.

### Preparation Example 16. Preparation of Comparative Compound 2 (AOH-1996)

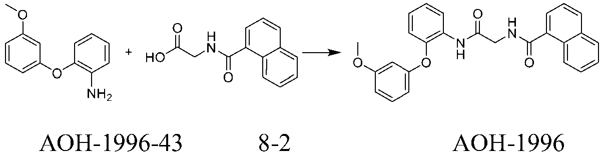

Compound **AOH-1996-43** (2.0 g, 9.29 mmol), compound **8-2** (2.6 g, 11.15 mmol), and HATU (5.3 g, 13.94 mmol) were dissolved in DMF (25 mL). Thereafter, DIPEA (4.9 mL, 27.87 mmol) was added dropwise. The mixture was reacted at room temperature for 16 h under nitrogen protection. The reaction solution was extracted with EA (100 mL), dried over anhydrous sodium sulfate, filtered, concentrated, purified by silica gel column chromatography (EA:petroleum ether=1:10-1:2), and recrystallized (EA/petroleum ether) to yield compound AOH-1996 (630 mg, 16%) as an off-white solid.

¹H NMR (400 MHz, CDCl₃) δ 8.42 (d, *J =* 7.4 Hz, 2H), 8.38 - 8.31 (m, 1H), 7.93 (d, *J* = 8.3 Hz, 1H), 7.89 - 7.82 (m, 1H), 7.61 (d, *J =* 7.0 Hz, 1H), 7.56 - 7.44 (m, 2H), 7.44 - 7.37 (m, 1H), 7.21 (t, *J* = 8.5 Hz, 1H), 7.13 (dd, *J* = 11.3, 4.2 Hz, 1H), 7.05 (t, *J* = 7.2 Hz, 1H), 6.92 (d, *J* = 7.2 Hz, 1H), 6.79 (s, 1H), 6.67 (dd, *J =* 8.9, 1.8 Hz, 1H), 6.61 - 6.53 (m, 2H), 4.36 (d, *J* = 5.5 Hz, 2H), 3.72 (s, 3H).m/z(ESI) [M+H]⁺=427.2.

### Bioassays

### 2.1 Antitumor Activity Assay

### 2.1.1 Cell Culture

### 2.1.2 Cell Culture Medium and Seeding Density

**Table 2 Cell Culture Conditions**

| **Cell Lines** | **Cell Culture** | **Seeding Density (cells/well)** |
|---|---|---|
| HL-60 | IMDM+20% FBS | 3200 |
| NCI-H522 | 1640+10% FBS | 1600 |

### 2.1.3 Cell Thawing and Recovery

a) The cell cryopreservation tube was taken out from liquid nitrogen and placed in a water bath at 37°C for rapid thawing. After the surface of the cryopreservation tube was wiped with 75% ethanol, the cryopreservation tube was transferred into a biosafety cabinet.
b) The cryopreserved cells were transferred into a 15-mL sterile centrifuge tube. Culture medium in a volume 10 times that of the cryopreservation solution was added, and centrifuged at 1,200 rpm for 5 min.
c) The culture medium in the centrifuge tube was discarded. An appropriate volume of fresh culture medium (Table 2) was added. The cells were re-suspended, transferred into a culture flask and cultured in a cell culture incubator at 37°C. The culture medium was replaced the next day.

### 2.1.4 Cell Passaging

### Adherent Cells:

a) For adherent cells at the logarithmic growth phase, the culture medium was discarded. The cells were washed with 8 mL of PBS two times, and the PBS was discarded.
b) 5 mL of a dissociation solution containing 0.25% trypsin was added, and the cells were incubated at 37°C for 3 to 5 min. The cells were observed under a microscope and found that a majority of the cells had become rounded.
c) 10 mL of culture medium was added to terminate the dissociation. The cells were gently pipetted to detach them from the culture surface, thereby obtaining a cell suspension for subsequent passaging and experimentation.

### Suspension Cells:

a) The cells were harvested directly and collected by centrifugation.

### 2.1.5 Cell Plating

a) Preparation of cell suspension
   i. the culture medium was removed from the culture flask;
   ii. the cells were rinsed with PBS two times;
   iii. trypsin was added to dissociate the cells, and after the dissociation was terminated by adding culture medium, the cells were collected by centrifugation; the suspension cells were collected directly;
   iv. the cells were re-suspended in culture medium, counted and adjusted to an appropriate concentration.
b) The cell suspension was added to a 384-well plate at a volume of 40 µL per well.
   The number of cells per well was shown in Table 2.
c) The cells were incubated at 37°C in a 5% CO₂ incubator for 48 h.

### 2.2 Treatment with Compound

### 2.2.1 Dilution of Compound

a) Preparation of gradient dilution solutions of positive compounds and test compounds:
   A 10 mM stock solution of each compound was prepared using DMSO. Subsequently, a 3-fold serial gradient dilution was carried out with DMSO to generate a total of 10 concentration points for each compound.

### 2.2.2 Cell Seeding

a) 40 nL of compound was transferred using an Echo550 system. The cell suspension was added to the cell culture plate at a volume of 40 µL per well.
b) The cells were transferred to an incubator and incubated for 48 h.

### 2.3 CTG Assay

a) The cell culture plate was removed and allowed to equilibrate at room temperature for 30 min.
b) The working solution for the CTG assay was prepared according to the instructions of the Promega kit and equilibrated to 25°C in an incubator.
c) 30 µL of CellTiter-Glo was transferred to each well of the cell culture plate. After incubation at room temperature for 15 min, the chemiluminescence was read using Ensight.

### 2.4 Data Analysis

The inhibition ratio (% inhibition) was calculated using the following formula: $% Inhibition = 100 % - 100 % \times \frac{{Lum}_{Sample} - {Lum}_{LC}}{{Lum}_{HC} - {Lum}_{LC}}$
Lum_{HC}: reading of cells in the control group treated with 1‰ DMSO
Lum_{Sample}: reading of cells treated with compound
Lum_{LC}: reading of cells in blank culture medium

The IC₅₀ was calculated using XLfit (5.3.1.3) by fitting the data to formula 201: y = (A+((B-A)/(1+((x/C)^D))))
A: Bottom
B: Top
C: IC₅₀ (Rel IC50)
D: Hill Slope

**Table 3 Inhibitory Activity of Compounds Against HL-60**

| **Compounds** | **Max. Concentration (µM)** | **IC₅₀ (µM)** |
|---|---|---|
| Compound 8 (MTB-1956) | 10 | 0.20962 |
| Compound 15 (MTB-1958) | 10 | 0.48445 |
| Compound 20 (MTB-1965) | 10 | 0.58106 |
| Compound 23 (MTB-1957) | 10 | 0.59291 |
| Compound 24 (MTB-1960) | 10 | 0.52759 |
| Compound 44 (MTB-1970) | 10 | 0.06735 |
| Compound 90 | 10 | 0.06801 |
| Compound 93 (MTB-1993) | 10 | 0.06304 |
| Compound 99 | 10 | 0.06401 |
| Compound 100 (MTB-2006) | 10 | 0.06308 |
| Comparative compound 1 (MTB-1962) | 10 | 6.85848 |
| Comparative compound 2 (AOH-1996) | 10 | 0.63189 |

The test results showed that the antitumor (HL-60) activity of the compound was significantly enhanced by substituting the methoxyphenyl group of Comparative Compound **2** (AOH-1996) with a specific bicyclic ring or further substituting the O atom between the two phenyl rings with an S atom. For example, Compound **8** (MTB-1956) exhibited three times higher inhibitory activity against HL-60 as compared to Comparative Compound **2** (AOH-1996), and compounds **44, 90,** and **93** exhibited approximately nine times higher inhibitory activity against HL-60 as compared to Comparative Compound **2** (AOH-1996). In contrast, Comparative Compound **1** (MTB-1962) obtained by substituting the methoxyphenyl group of Comparative Compound **2** (AOH-1996) with a benzothiazole bicyclic ring showed a significantly reduced activity, which was 11 times lower than that in Comparative Compound **2** (AOH-1996). The para position of an amino group was generally susceptible to oxidation by P450, and thus fluorine substitution could improve its biological stability. The test results demonstrated that the antitumor activity (HL-60) of the fluorinated compound 99 and its methyl-deuterated compound 100 was comparable to that of the corresponding compounds **44** and **93.**

**Table 4 Inhibitory Activity of Compounds Against NCI-H522**

| **Compound** | **Max. Concentration (µM)** | **IC₅₀ (µM)** |
|---|---|---|
| Compound 8 (MTB-1956) | 10 | 0.13433 |
| Comparative compound 1 (MTB-1962) | 10 | 2.04096 |
| Comparative compound 2 (AOH-1996) | 10 | 0.23399 |

The test results showed that the antitumor activity (NCI-H522) was significantly enhanced by substituting the methoxyphenyl group of AOH-1996 with a specific bicyclic ring, such as Compound **8** (MTB-1956). In contrast, Comparative Compound **1** obtained by substituting the methoxyphenyl group of AOH-1996 with a benzothiazole bicyclic ring exhibited a lower activity.

### Broad-Spectrum Antitumor Activity Assay:

According to the test methods described above, the antitumor activities of the test compounds and AOH-1996 in various tumor cell lines were tested (Table 5).

| Tumor Cell Lines | Compounds | IC₅₀ |
|---|---|---|
| **colon cancer (HCT-116) IC₅₀ (µM)** | Comparative compound 2 (AOH-1996) | 0.22018 |
| | Compound 44 (MTB-1970) | 0.02576 |
| | Compound 93 (MTB-1993) | 0.02052 |
| **leukemia (HL-60) IC₅₀ (µM)** | Comparative compound 2 (AOH-1996) | 0.38492 |
| | Compound 44 (MTB-1970) | 0.06257 |
| | Compound 93 (MTB-1993) | 0.06459 |
| | Compound 99 | 0.08105 |
| | Compound 100 (MTB-2006) | 0.07857 |
| Human renal cancer **(786-O) IC₅₀ (µM)** | Comparative compound 2 (AOH-1996) | 0.31872 |
| | Compound 44 (MTB-1970) | 0.06342 |
| | Compound 93 (MTB-1993) | 0.06315 |
| | Compound 99 | 0.07906 |
| | Compound 100 (MTB-2006) | 0.07822 |
| Non-small cell lung cancer **(A-549) IC₅₀ (µM)** | Comparative compound 2(AOH-1996) | 0.73040 |
| | Compound 44 (MTB-1970) | 0.11303 |
| | Compound 93 (MTB-1993) | 0.07193 |
| Human glioma **(U-251) IC₅₀(µM)** | Comparative compound 2 (AOH-1996) | 0.33158 |
| | Compound 44 (MTB-1970) | 0.06646 |
| | Compound 93 (MTB-1993) | 0.06084 |
| Human prostate cancer **(PC-3) IC₅₀ (µM)** | Comparative compound 2 (AOH-1996) | 0.35106 |
| | Compound 44 (MTB-1970) | 0.07959 |
| | Compound 93 (MTB-1993) | 0.06705 |
| Human ovarian cancer **(OVCAR-3) IC₅₀ (µM)** | Comparative compound 2 (AOH-1996) | 0.53611 |
| | Compound 44 (MTB-1970) | 0.15372 |
| | Compound 93 (MTB-1993) | 0.08030 |
| | Compound 99 | 0.08820 |
| | Compound 100 (MTB-2006) | 0.08625 |
| Human breast cancer **(MCF7) IC₅₀ (µM)** | Comparative compound 2 (AOH-1996) | 0.46922 |
| | Compound 44 (MTB-1970) | 0.09138 |
| | Compound 93 (MTB-1993) | 0.07253 |
| | Compound 99 | 0.07928 |
| | Compound 100 (MTB-2006) | 0.08132 |
| **Human peripheral blood mononuclear cell (Human PBMC) IC₅₀ (µM)** | Comparative compound 2 (AOH-1996) | >10 |
| | Compound 44 (MTB-1970) | >10 |
| | Compound 93 (MTB-1993) | >10 |
| | Compound 99 | >10 |
| | Compound 100 (MTB-2006) | >10 |

The test results showed that compound 44 (MTB-1970), compound 99, compound 93 (MTB-1993) (a methyl-deuterated product of compound 99), and compound 100 (MTB-2006) exhibited inhibitory activities against all test tumor cell lines that were 5 to 10 times higher than that of AOH-1996, and showed no significant cytotoxicity toward normal human peripheral blood mononuclear cells (PBMCs) at the highest test concentration of 10 µM, demonstrating great potential for development. Compound 100 (MTB-2006), which was obtained by substituting the hydrogen atom at the para position of the amino group of compound 93 (MTB-1993) with a fluorine atom, exhibited an antitumor activity comparable to that of compound 93 (MTB-1993).

### Pharmacokinetic Experiments

### Pharmacokinetic studies on administration of comparative compound 2 (AOH-1996), compound 100 (MTB-2006) and compound 99 via single gavage to female CB-17 SCID (B-Ces1c KO) mice

### 1. Research Methods and Experimental Design

### 1.1. Experimental System

| | |
|---|---|
| species | mouse |
| strain | CB-17 SCID (B-Ces1c KO) |
| source | Biocytogen |
| sex and number | 12 rats (12 male) |
| age at the start of test | 5 to 8 weeks |

### 1.2. Experimental Design

Prior to the first administration, animals were grouped based on body weight. The animals were weighed before administration, and the dosing volume was calculated based on the body weight.

### 2. Sample Collection

### 2.1. Formulation for Administration

After each formulation was prepared, two aliquots of the clear dosing solution (top and bottom layers) or three aliquots of the suspension (top, middle, and bottom layers) were collected. The formulation was analyzed by HPLC-UV to evaluate the accuracy of the formulation concentration.

### 2.2. Sample Collection and Processing

Plasma samples were collected at 0.167, 0.333, 0.5, 1, 2, 4, 6, and 12 h after administration.

**Whole blood collection and preparation of plasma samples:** Whole blood was collected from the saphenous vein of each animal (approximately 0.02 mL per time point) into pre-cooled commercial blood collection tubes (containing EDTA-K2 as an anticoagulant) at each time point. Within 10 seconds after collection of the whole blood, a stabilizer was added at a ratio of whole blood to 400 mM DDV (19:1, v:v). The samples were placed on wet ice till centrifugation. Blood samples collected at each time were kept on wet ice prior to centrifugation, and centrifuged at approximately 4°C and 3,200 g for 10 min to prepare plasma samples. Subsequently, the plasma samples were transferred to low-adsorption tubes, flash-frozen on dry ice, and stored at a temperature of -60°C or lower till LC-MS/MS. The sample processing was carried out under wet ice conditions.

### 3. Sample Analysis

### 3.1. Formulation for Administration

The concentration of the formulation for administration was determined by the HPLC-UV method. The calibration curve consisted of at least six concentration levels. Each sample was analyzed in duplicate. The measured concentration should fall within 80% to 120% of the theoretical formulation concentration; otherwise, the pharmacokinetic parameters should be calculated based on the actual dose.

### 3.2. Bioanalytical Method Development and Sample Analysis

Under non-GLP conditions, an LC-MS/MS method for the quantitative determination of the test compounds in biological matrices was established.

**Table 6. Pharmacokinetic Data of AOH-1996, Compound 99, and MTB-2006**

| | AOH-1996 | Compound 99 | MTB-2006 |
|---|---|---|---|
| AUC 0-last (ng.h/mL) | 162 | 345 | 457 |
| Cmax (ng/mL) | 54.3 | 68.6 | 98.5 |
| T_{1/2} (h) | 2.99 | 3.21 | 3.73 |

The pharmacokinetic results (Table 6) suggested that compound 99 and compound 100 (MTB-2006) showed better biological stability, and the methyl-deuterated compound showed further improved biological stability. The drug exposure of compound **MTB-2006** was approximately three times that of **AOH-1996.** The drug exposure of deuterated compound **MTB-2006** was also significantly increased as compared to compound 99 (32.5%). Both MTB-2006 and compound 99 showed significantly improved Cₘₐₓ and half-life as compared to compound AOH-1996, with MTB-2006 showing better performance.

As could be seen from the above experimental results, the exemplary compounds exhibited 5- to 10-fold higher *in vitro* inhibitory activities against various tumor cells as compared to Comparative Compound 2 **(AOH-1996),** along with significantly improved biological stability and drug exposure. These results demonstrated their greater development potential and clinical value.

The specific embodiments of the present disclosure have been described above by way of example. However, the present disclosure is not limited to the specific embodiments described above. Any modifications, equivalent substitutions, etc. made within the spirit and principles of the present disclosure shall be encompassed within the scope of the present disclosure.

## Claims

1. A compound represented by formula (I), or a pharmaceutically acceptable salt thereof, or a deuterated compound thereof, 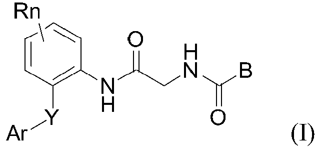 wherein:
B is substituted or unsubstituted naphthyl, substituted or unsubstituted quinolinyl, or substituted or unsubstituted isoquinolinyl; optionally, B is substituted or unsubstituted naphthyl; optionally, B is naphthyl; optionally, substituents in the substituted naphthyl, the substituted quinolinyl, and the substituted isoquinolinyl are each independently C₁₋₆ alkyl, C₃₋₇ cycloalkyl, C₆₋₁₀ aryl, halogen, or halogenated C₁₋₆ alkyl; optionally, the substituents are each independently methyl, ethyl, n-propyl, isopropyl, cyclopropyl, phenyl, F, CI, Br, I, or -CF₃;
each R is independently C₁₋₆ alkyl, C₃₋₇ cycloalkyl, substituted or unsubstituted C₆₋₁₀ aryl, substituted or unsubstituted 5- to 10-membered heteroaryl containing 1 to 3 heteroatoms selected from N, O, and S, -NH₂, -CN, halogen (such as F, Cl, Br, I), -OH, or -OR', wherein R' is C₁₋₆ alkyl, C₃₋₇ cycloalkyl, or substituted or unsubstituted C₆₋₁₀ aryl; optionally, the alkyl is methyl, ethyl, n-propyl, or isopropyl; the cycloalkyl is cyclopropyl; the substituents in the substituted aryl and the substituted heteroaryl are each independently C₁₋₆ alkyl, C₃₋₇ cycloalkyl, C₆₋₁₀ aryl, halogen, or halogenated C₁₋₆ alkyl; optionally, the substituents are each independently methyl, ethyl, n-propyl, isopropyl, cyclopropyl, phenyl, F, CI, Br, I, or -CF₃;
n is an integer selected from 0 to 3; optionally, n is 0;
Y is O or S; preferably, Y is O; preferably, Y is S;
Ar is carbocyclic ring-fused phenyl, heterocyclic ring-fused phenyl, carbocyclic ring-fused heteroaryl, or heterocyclic ring-fused heteroaryl; optionally, Ar is carbocyclic ring-fused phenyl or heterocyclic ring-fused phenyl; the carbocyclic ring is a saturated or non-aromatic unsaturated 5-membered or 6-membered carbocyclic ring, the heterocyclic ring is a 5-membered or 6-membered heterocyclic ring containing 1 or 2 N atoms, 1 or 2 O atoms, or 1 or 2 S atoms on a saturated or non-aromatic unsaturated ring, and the heteroaryl is a 6-membered heteroaryl containing 1 or 2 N atoms on the ring; optionally, Ar is heterocyclic ring-fused phenyl, and the heterocyclic ring is a 5- or 6-membered heterocyclic ring containing 1 or 2 O atoms on a saturated or non-aromatic unsaturated ring;
the carbocyclic ring-fused phenyl, the heterocyclic ring-fused phenyl, the carbocyclic ring-fused heteroaryl, and the heterocyclic ring-fused heteroaryl are optionally each independently substituted by one or more substituents, and the substituents are each independently C₁₋₆ alkyl, C₃₋₇ cycloalkyl, OH, CN, NH₂, mono- or di-C₁₋₆ alkylamino, halogen, or halogenated C₁₋₆ alkyl; optionally, the carbocyclic ring-fused phenyl, the heterocyclic ring-fused phenyl, the carbocyclic ring-fused heteroaryl, and the heterocyclic ring-fused heteroaryl are optionally each independently substituted by one or more substituents, and the substituents are each independently methyl, ethyl, n-propyl, isopropyl, cyclopropyl, -F, -Cl, -Br, -I, -CH₂CF₃, or -CF₃; optionally, the substituents are each independently -CH₃, -F, -Cl, or -CF₃.

2. The compound represented by formula (I), or the pharmaceutically acceptable salt thereof, or the deuterated compound thereof according to claim 1, wherein
Ar is selected from the following groups: 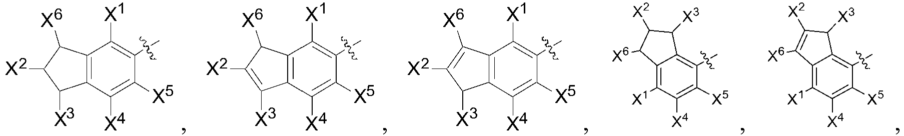 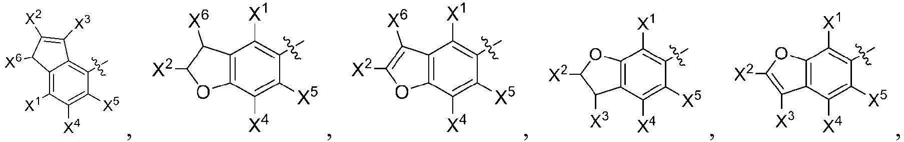 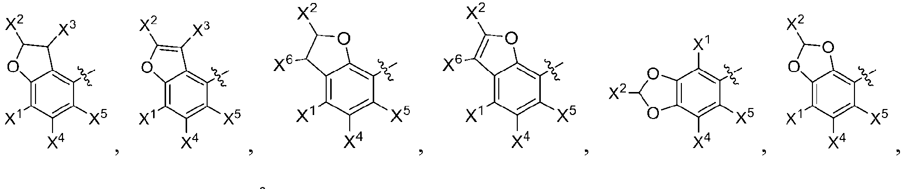 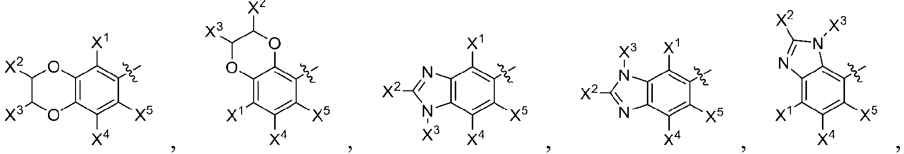 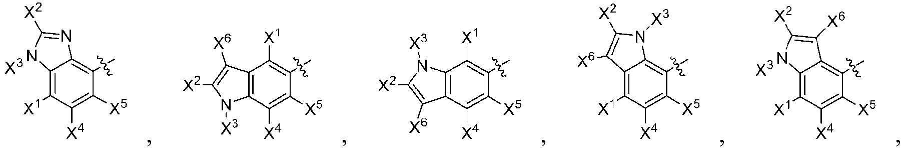 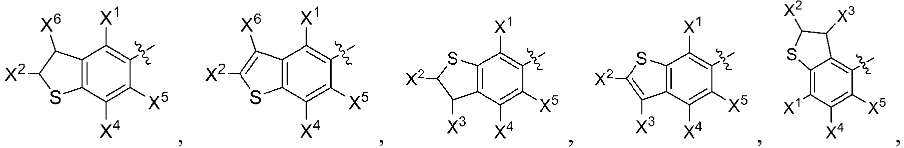 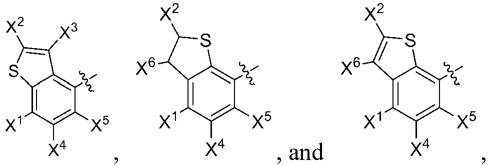
wherein X¹, X², X³, X⁴, X⁵, and X⁶ are each independently H, C₁₋₆ alkyl (such as methyl, ethyl, n-propyl, or isopropyl), C₃₋₇ cycloalkyl (such as cyclopropyl), OH, CN, NH₂, halogen (such as Cl, Br, I, or F), or halogenated C₁₋₆ alkyl; optionally, X¹, X⁴, and X⁵ are each independently H, methyl, ethyl, n-propyl, isopropyl, cyclopropyl, -F, -Cl, -Br, -I, -CH₂CF₃, or -CF₃, preferably X¹ and/or X⁴ and/or X⁵ is hydrogen; optionally, X¹, X⁴, and X⁵ are each independently H, -CH₃, -F, -Cl, or -CF₃, preferably X¹ and/or X⁴ and/or X⁵ is hydrogen; optionally, X², X³, and X⁶ are each independently H, methyl, ethyl, n-propyl, isopropyl, cyclopropyl, -F, -Cl, -Br, -I, -CH₂CF₃, or -CF₃, preferably X² and/or X³ and/or X⁶ is hydrogen; optionally, X², X³, and X⁶ are each independently H or -CH₃, preferably two or all of X², X³, and X⁶ are hydrogen.

3. The compound represented by formula (I), or the pharmaceutically acceptable salt thereof, or the deuterated compound thereof according to claim 1 or 2, wherein
Ar is selected from the following groups: 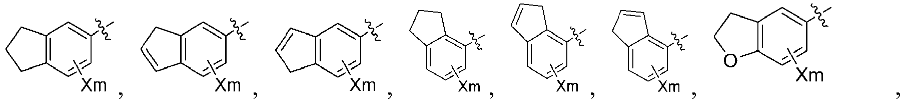 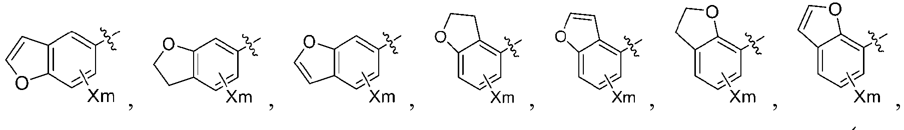 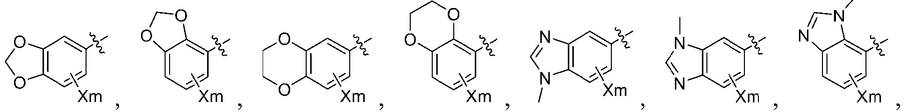 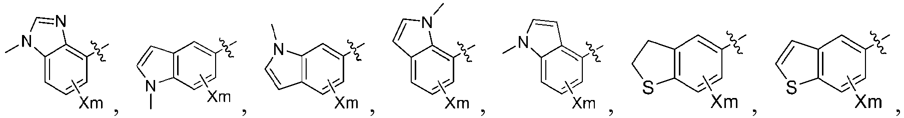 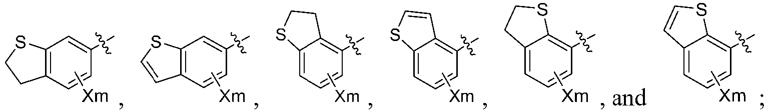
wherein each X is independently C₁₋₆ alkyl (such as methyl, ethyl, n-propyl, or isopropyl), C₃₋₇ cycloalkyl (such as cyclopropyl), OH, CN, NH₂, halogen (such as Cl, Br, I, or F) or halogenated C₁₋₆ alkyl; optionally, each X is independently methyl, ethyl, n-propyl, isopropyl, cyclopropyl, F, Cl, Br, I, -CH₂CH₃, or -CF₃; optionally, each X is independently -CH₃, -F, -Cl, or -CF₃;
m is an integer of from 0 to 3; optionally, m is 0, 1 or 2.

4. The compound represented by formula (I), or the pharmaceutically acceptable salt thereof, or the deuterated compound thereof according to any one of claims 1 to 3, wherein
the compound represented by formula (I) is selected from the following compounds: 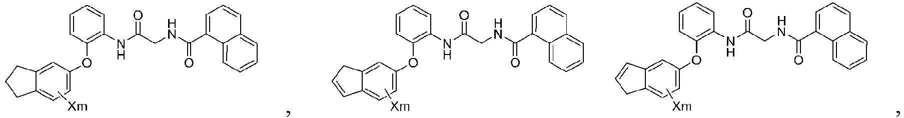 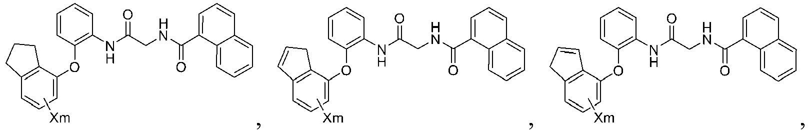 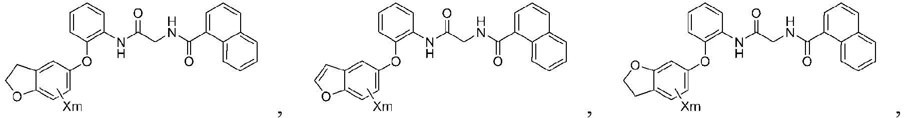 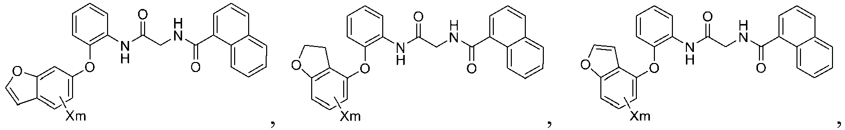 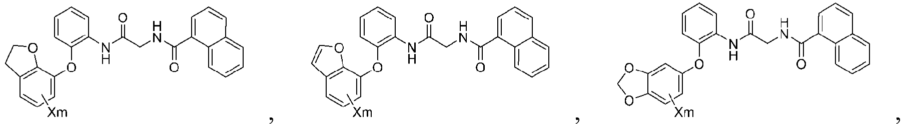 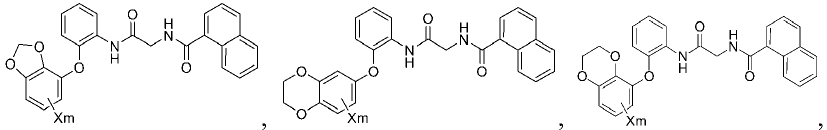 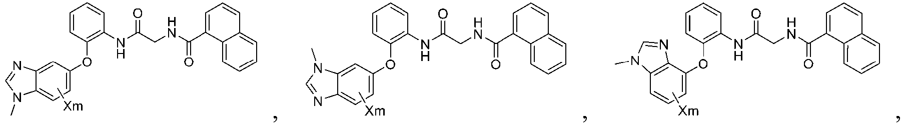 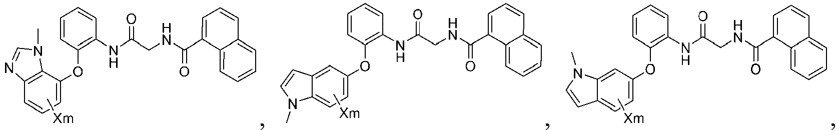 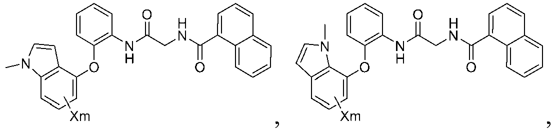 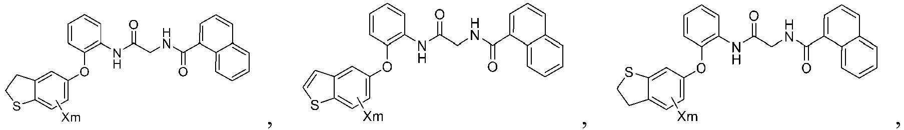 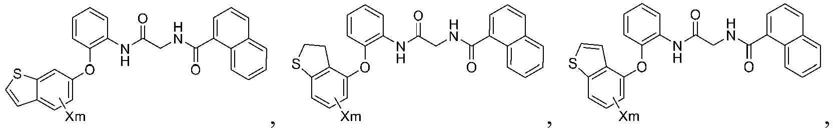 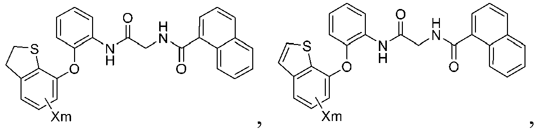 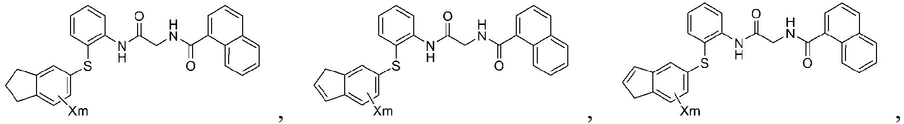 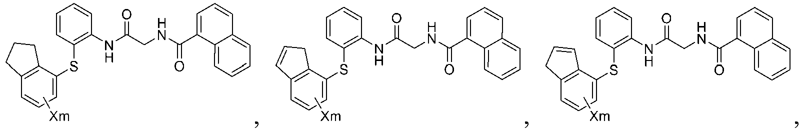 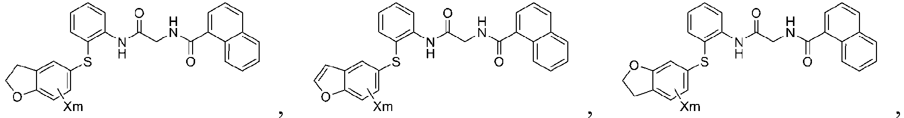 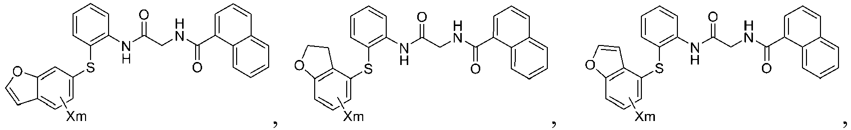 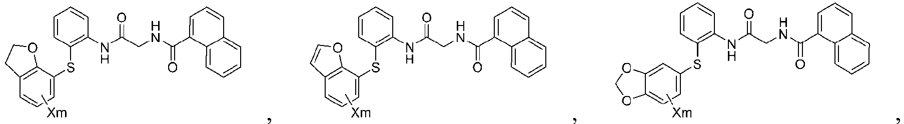 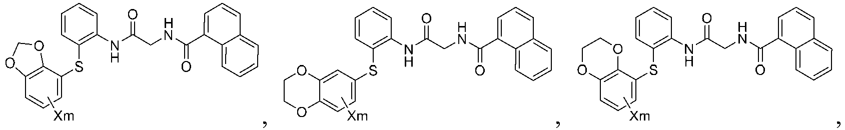 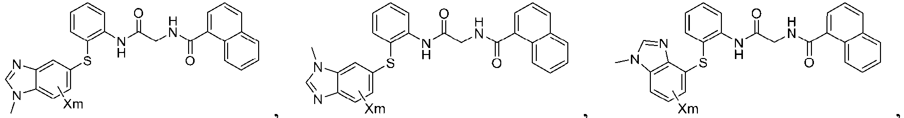 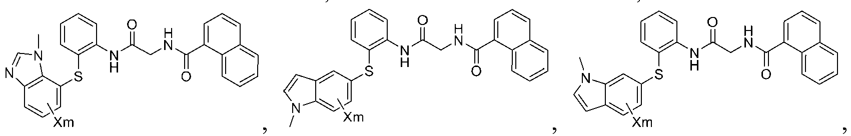 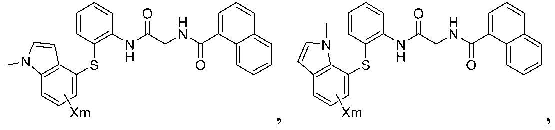 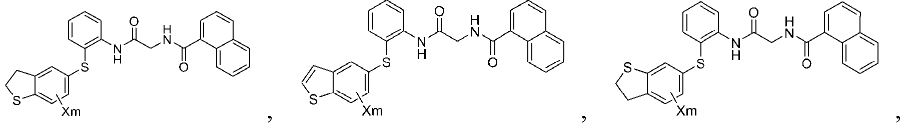 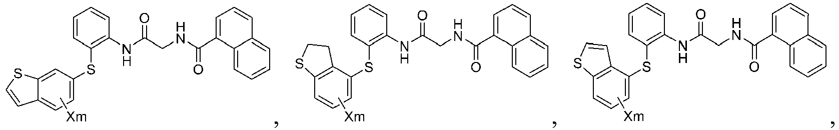 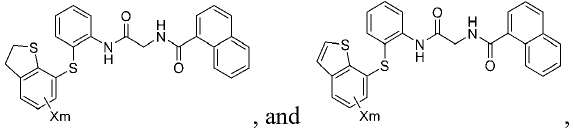
wherein each X is independently C₁₋₆ alkyl (such as methyl, ethyl, n-propyl, or isopropyl), C₃₋₇ cycloalkyl (such as cyclopropyl), -OH, -CN, -NH₂, halogen (such as Cl, Br, I, or F), or halogenated C₁₋₆ alkyl; optionally, each X is independently methyl, ethyl, propyl, n-propyl, isopropyl, cyclopropyl, -F, -Cl, -Br, -I, -CH₂CH₃, or -CF₃; optionally, each X is independently -CH₃, -F, -Cl, or -CF₃;
m is an integer of from 0 to 3; optionally, m is 0, 1 or 2.

5. The compound represented by formula (I), or the pharmaceutically acceptable salt thereof, or the deuterated compound thereof according to claim 1, wherein
the compound represented by formula (I) or the deuterated compound thereof is selected from the following compounds: 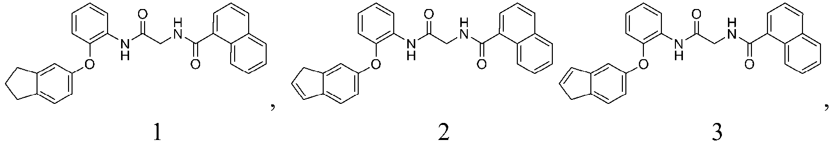 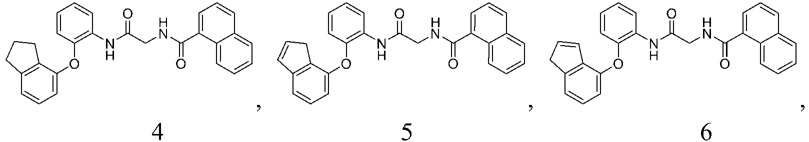 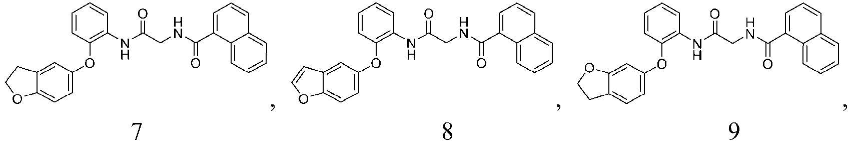 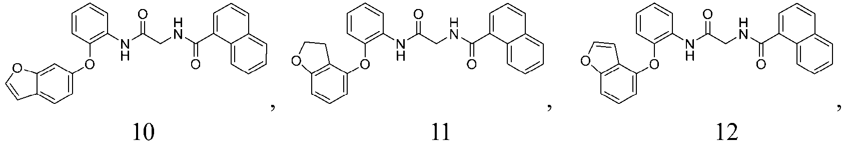 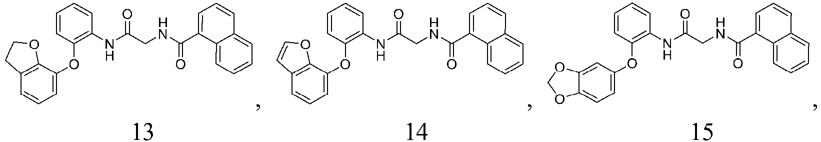 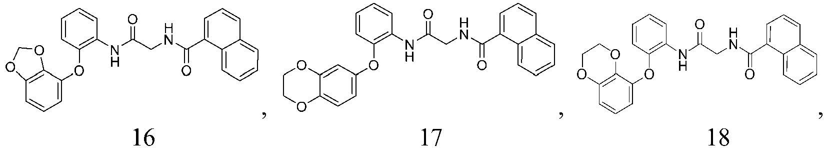 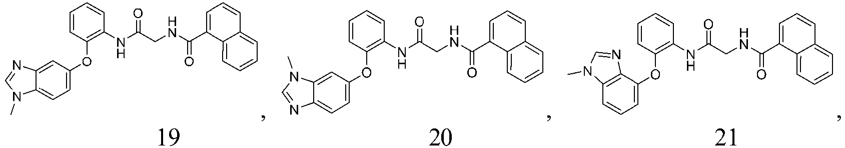 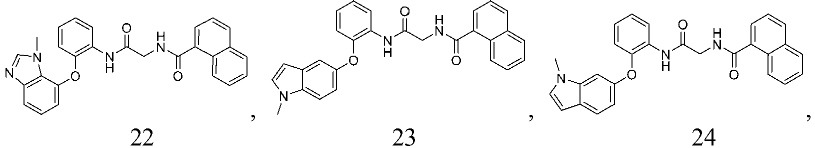 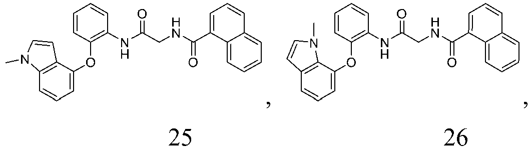 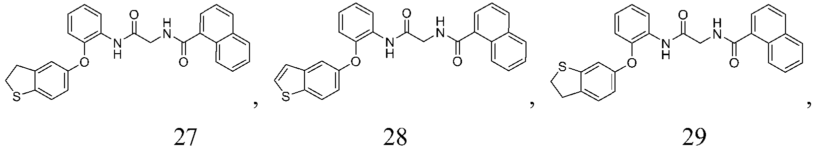 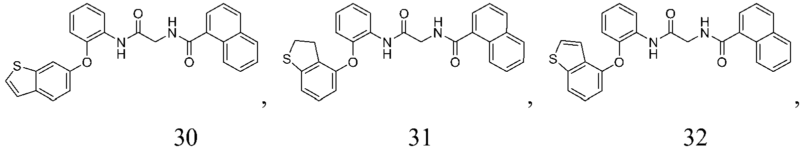 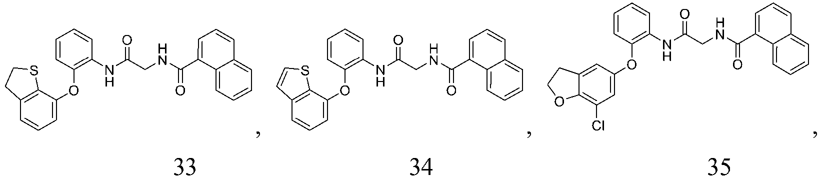 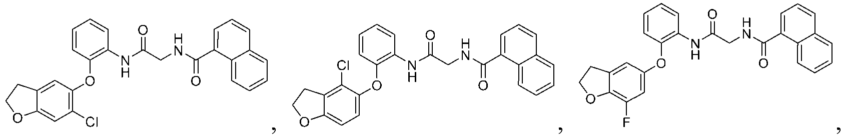 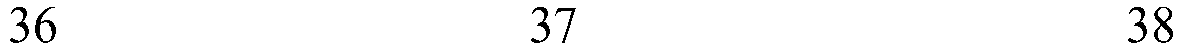 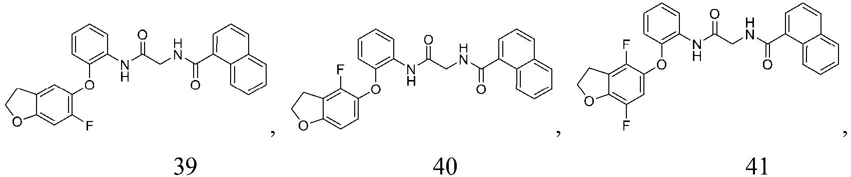 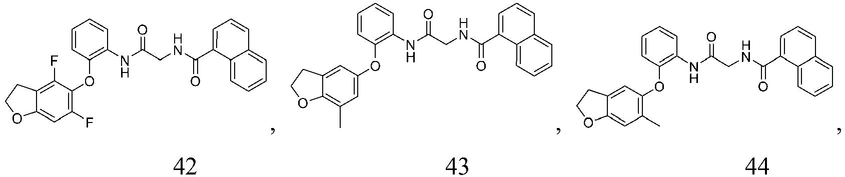 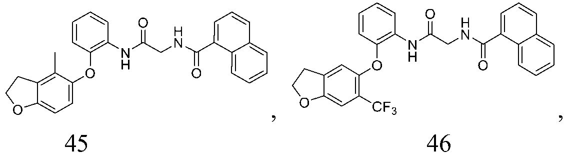 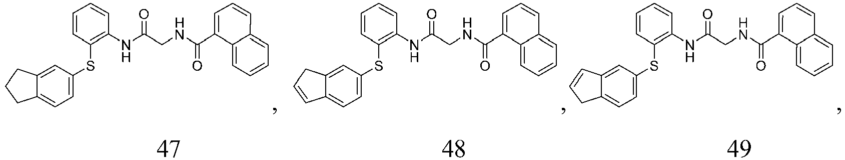 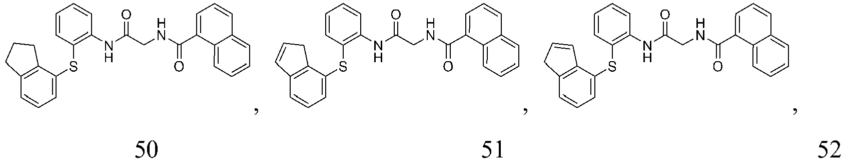 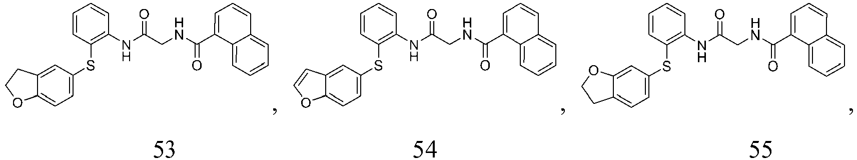 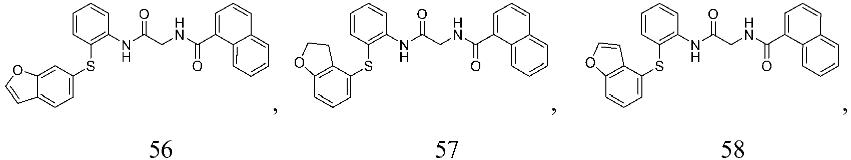 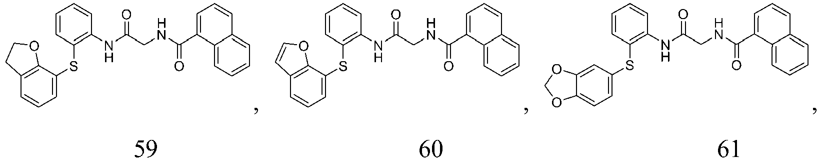 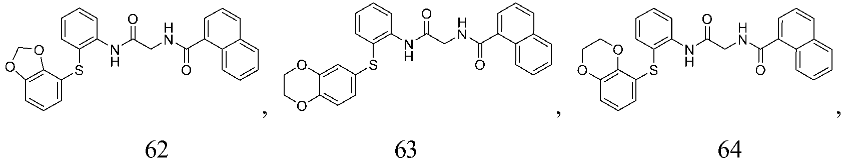 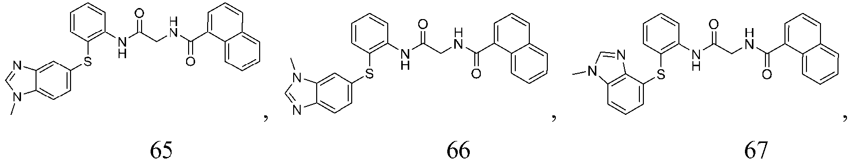 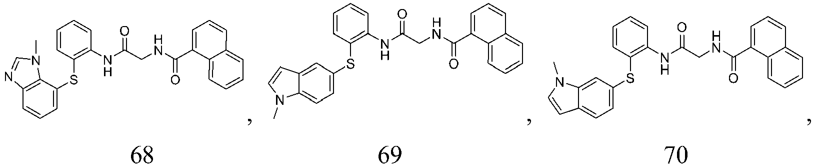 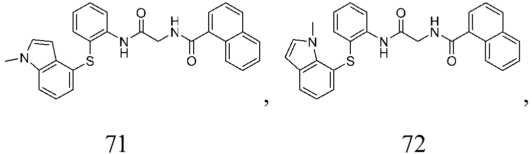 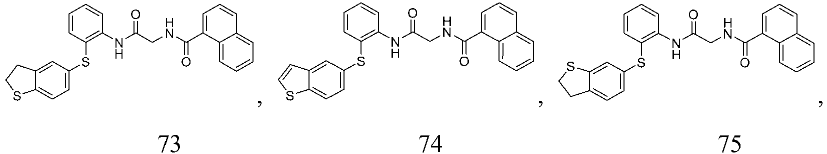 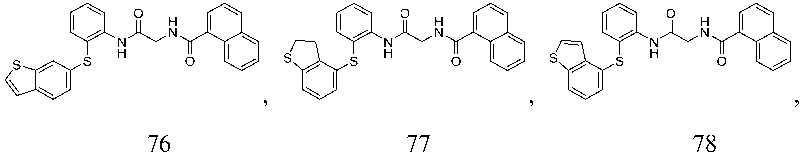 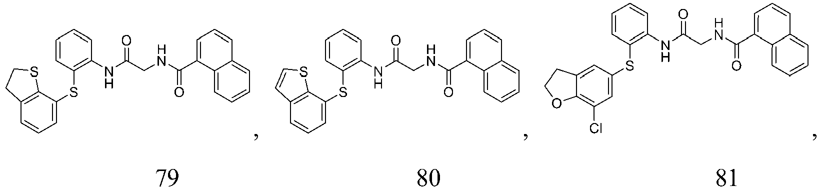 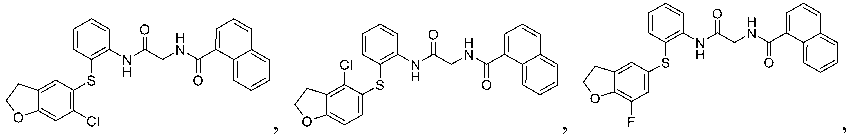 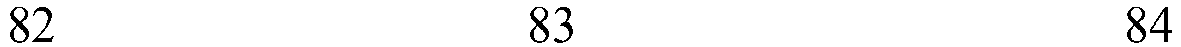 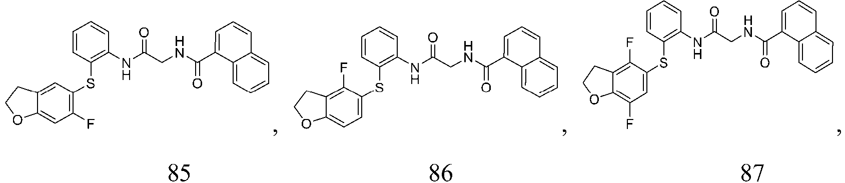 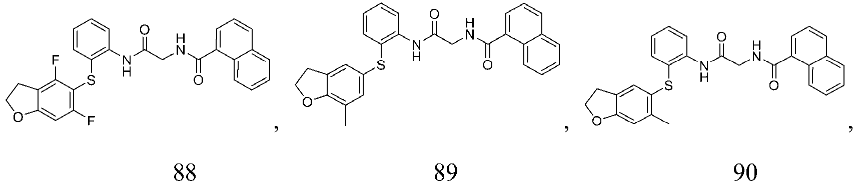 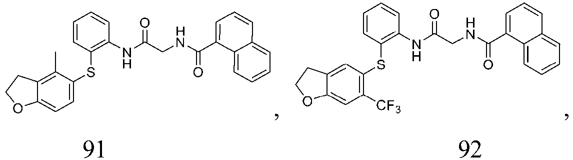 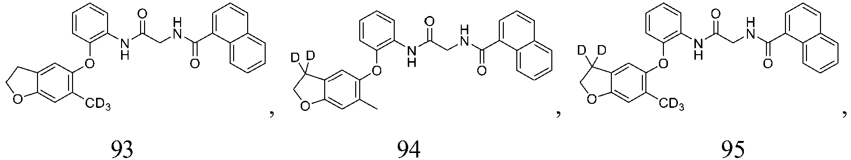 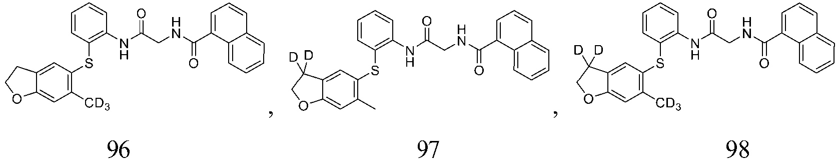 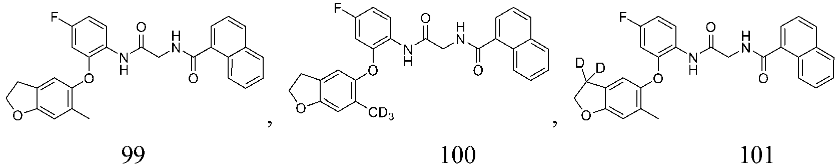 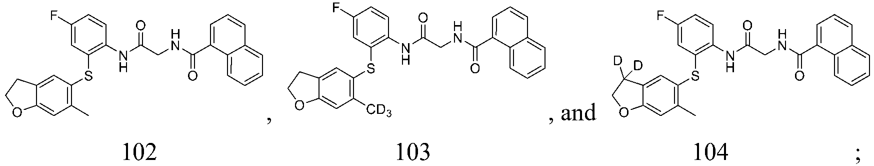 particularly preferably compound 44, compound 93, compound 99 or compound 100.

6. The compound represented by formula (I), or the pharmaceutically acceptable salt thereof, or the deuterated compound thereof according to any one of claims 1 to 5, wherein
the pharmaceutically acceptable salt is a salt formed by the compound represented by formula (I) or the deuterated compound thereof with an acid; the acid includes an inorganic acid or an organic acid; optionally, the inorganic acid includes hydrochloric acid, hydrobromic acid, sulfuric acid, nitric acid, phosphoric acid, or carbonic acid; optionally, the organic acid includes formic acid, acetic acid, propionic acid, oxalic acid, malonic acid, succinic acid, phthalic acid, fumaric acid, maleic acid, lactic acid, malic acid, citric acid, tartaric acid, carbonic acid, picric acid, methanesulfonic acid, ethanesulfonic acid, benzenesulfonic acid, or p-toluenesulfonic acid.

7. A method for preparing the compound represented by formula (I), or the pharmaceutically acceptable salt thereof, or the deuterated compound thereof according to any one of claims 1 to 6, wherein the method comprises the following reaction route: 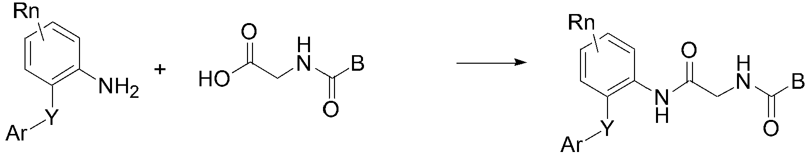 Ar, Y, R, n, and B in structural formulae described above are as defined in any one of claims 1 to 6.

8. An intermediate compound as shown below:

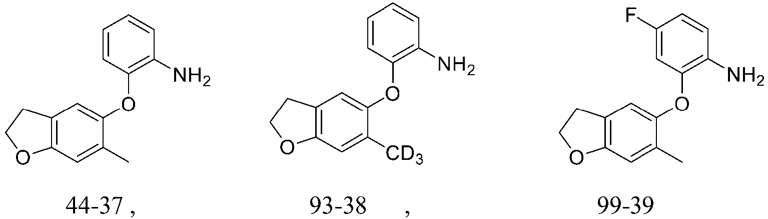

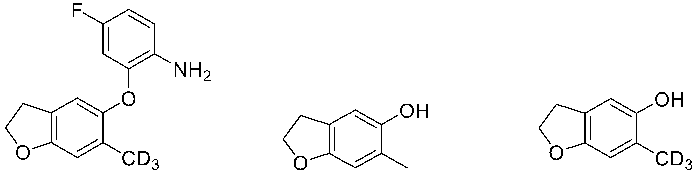

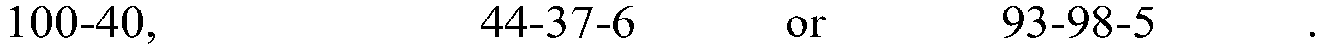

9. A pharmaceutical composition, comprising one or more of the compound represented by formula (I), or the pharmaceutically acceptable salt thereof, or the deuterated compound thereof according to any one of claims 1 to 6, and optionally a pharmaceutically acceptable excipient.

10. The pharmaceutical composition according to claim 9, further comprising an anticancer drug in addition to the compound represented by formula (I), or the pharmaceutically acceptable salt thereof, or the deuterated compound thereof; preferably, the anticancer drug comprising a chemotherapeutic drug, or/and a targeted drug, or/and a radiopharmaceutical; preferably, the anticancer drug comprising a chemotherapeutic drug such as cyclophosphamide; an antimetabolite drug such as fluorouracil, gemcitabine, or capecitabine; an antibiotic such as doxorubicin; a platinum-based anticancer drug such as carboplatin, cisplatin, or oxaliplatin; a phytopharmaceutical drug such as irinotecan, paclitaxel, or taxotere; a targeted drug such as afatinib, trastuzumab, imatinib, panitumumab, ramucirumab, or osimertinib; an immune preparation such as pembrolizumab, nivolumab, or Keytruda; and an ADC-based drug such as gemtruzumab, trastuzumab emtansine, sacituzumab tirumotecan, trastuzumab deruxtecan, or Enherut; a bispecific antibody drug such as PD-1/CTLA-4 bispecific antibody cadonilimab or PD-(L)1/VEGF bispecific antibody ivonescimab, and a radiopharmaceutical such as lutetium[177Lu] vipivotide tetraxetan injection (Pluvicto) or Lutathera.

11. Use of the compound represented by formula (I), or the pharmaceutically acceptable salt thereof, or the deuterated compound thereof according to any one of claims 1 to 6, or the pharmaceutical composition according to claim 9 in the preparation of a PCNA inhibitor.

12. Use of the compound represented by formula (I), or the pharmaceutically acceptable salt thereof, or the deuterated compound thereof according to any one of claims 1 to 6, or the pharmaceutical composition according to claim 9 in the preparation of a medicament for preventing or treating cancer; wherein optionally, the cancer includes, but is not limited to, lung cancer, melanoma, colon cancer, rectal cancer, prostate cancer, ovarian cancer, and leukemia; preferably, the compound represented by formula (I), or the pharmaceutically acceptable salt thereof, or the deuterated compound thereof, or the pharmaceutical composition may also be used in combination with an anticancer drug in addition thereto; preferably, the anticancer drug comprises a chemotherapeutic drug, or/and a targeted drug, or/and a radiopharmaceutical; preferably, the anticancer drug comprises a chemotherapeutic drug such as cyclophosphamide; an antimetabolite drug such as fluorouracil, gemcitabine, or capecitabine; an antibiotic such as doxorubicin; a platinum-based anticancer drug such as carboplatin, cisplatin, or oxaliplatin; a phytopharmaceutical drug such as irinotecan, paclitaxel, or taxotere; a targeted drug such as afatinib, trastuzumab, imatinib, panitumumab, ramucirumab, or osimertinib; an immune preparation such as pembrolizumab, nivolumab, or Keytruda; and an ADC-based drug such as gemtruzumab, trastuzumab emtansine, sacituzumab tirumotecan, trastuzumab deruxtecan, or Enherut; a bispecific antibody drug such as PD-1/CTLA-4 bispecific antibody cadonilimab or PD-(L)1/VEGF bispecific antibody ivonescimab, and a radiopharmaceutical such as lutetium[177Lu] vipivotide tetraxetan injection (Pluvicto) or Lutathera.
